# EUROPEAN PATENT SPECIFICATION

(11) **EP 4 079 334 B1**
(45) Date of publication and mention of the grant of the patent: **05.03.2025**
(21) Application number: 22166432.9
(22) Date of filing: 04.04.2022
(51) Int. Cl.: A61L 2/10, A45F 3/00, A61L 9/20, G02B 5/00

(54) **ADAPTABLE SANITIZING SYSTEMS**
ANPASSUNGSFÄHIGE DESINFEKTIONSSYSTEME
SYSTÈMES D'ASSAINISSEMENT ADAPTABLES

(30) Priority: 20.04.2021 US 202163176919 P
(43) Date of publication of application: 26.10.2022
(73) Proprietor: The Boeing Company, Arlington, VA 22202 (US)
(72) Inventor: CHILDRESS, Jamie J., CHICAGO, 60606-2016 (US)
(74) Representative: Plasseraud IP

(56) References cited:
- CN-U- 211 048 664
- JP-A- 2006 087 472
- KR-A- 20160 148 140
- US-A1- 2019 038 914

## Description

### FIELD OF THE DISCLOSURE

Examples of the present disclosure generally relate to adaptable sanitizing systems, such as may be used to sanitize structures and areas within vehicles, such as commercial aircraft.

### BACKGROUND OF THE DISCLOSURE

Vehicles such as commercial aircraft are used to transport passengers between various locations. Systems are currently being developed to disinfect or otherwise sanitize surfaces within aircraft, for example, that use ultraviolet (UV) light. In order to sanitize a surface of a structure, a known UV light sterilization method emits a broad spectrum UVC light onto the structure.

Known UV light sanitizing systems are typically configured for a particular application. As an example, a UV light sanitizing system emits UV light at a particular wavelength in order to sanitize a surface.

However, in certain settings, a different type of wavelength of UV light may be desired. Accordingly, a different UV lamp that emits UV light at a different wavelength is typically used.

KR20160148140, in accordance with its abstract, states a portable UV sterilizing device comprising: a substrate having a UV LED mounted thereon; a housing for accommodating the substrate; an optic for adjusting a diffusion angle of ultraviolet rays irradiated from the UV LED; and a connector electrically connected with the substrate, and serving as a route provided with electrical power from the outside. Moreover, this document provides a portable UV disinfecting device comprising: a substrate having a UV LED mounted thereon; a housing for accommodating the substrate; an optic for adjusting a diffusion angle of ultraviolet rays irradiated from the UV LED; and a battery electrically connected with the substrate, accommodated in the housing, and supplying electrical power to the UV LED.

US2019/038914, in accordance with its abstract, states a sterilization apparatus configured to irradiate a sterilization target organism on a body or in the body with light and thereby killing or inactivating the sterilization target organism. The sterilization apparatus includes: a light source configured to emit light having wavelengths within a wavelength range of 190 nm to 230 nm and a wavelength range of 230 to 300 nm; a power supply unit configured to supply power to the light source; a control unit configured to control the power supply unit; and an optical filter. The power supply unit is controlled by the control unit so that an irradiation amount of light having a wavelength within the wavelength range of 230 to 300 nm in one light irradiation is not more than 9 mJ/cm2.

JP2006087472, in accordance with its abstract, states an instrument for ultraviolet irradiation equipped with a plurality of light-shielding filters, each having different transmittances for ultraviolet ray emitted from a light source for irradiating a skin with ultraviolet radiation and a support member for supporting the light-shielding filters.

### SUMMARY OF THE DISCLOSURE

A need exists for a system and a method for adapting a UV light sanitizing system. Further, a need exists for a UV light sanitizing system and method that can be used with respect to various different applications. The invention is defined in the appended claims.

With those needs in mind, certain examples of the present disclosure provide an adaptable sanitizing system including a sanitizing head including an ultraviolet (UV) lamp configured to emit UV light. One or more adapter modules are configured to removably couple to the sanitizing head at a coupling interface. The one or more adapter modules are configured to provide a functionality in relation to the UV light emitted by the UV lamp. For example, the functionality includes one of optical filtering, optical wavelength converting, fluid sanitizing, or object sanitizing.

In at least one example, the one or more adapter modules include a first adapter module configured to perform a first unique functionality, and a second adapter module configured to perform a second unique functionality that differs from

the first unique functionality. The first adapter module and the second adapter module are interchangeable in relation to the sanitizing head. As an example, the first unique functionality includes one of optical filtering, optical wavelength converting, fluid sanitizing, or object sanitizing, and the second unique functionality comprises another of optical filtering, optical wavelength converting, fluid sanitizing, or object sanitizing.

In at least one example, the coupling interface is common to the first adaptable module and the second adapter module.

In at least one example, a wand assembly includes the sanitizing head. The wand assembly may be coupled to a backpack assembly. As another example, the wand assembly may be coupled to a case assembly.

Certain examples of the present disclosure provide an adaptable sanitizing method, comprising removably coupling one or more adapter modules to a sanitizing head at a coupling interface, wherein sanitizing head includes an ultraviolet (UV) lamp configured to emit UV light, and wherein the one or more adapter modules are configured to provide a functionality in relation to the UV light emitted by the UV lamp.

Certain examples of the present disclosure provide an adapter module configured to removably couple to a sanitizing head having an ultraviolet (UV) lamp that is configured to emit UV light. The adapter module includes a shroud or frame that is configured to removably couple to the sanitizing head at a coupling interface, and an optical filter coupled to the shroud or the frame. The optical filter is configured to filter the UV light emitted by the UV lamp. The adapter module is distinct from the sanitizing head.

As an example, the optical filter is a 230 nanometer low pass filter. In at least one example, the optical filter includes a panel secured to the shroud or the frame. As an example, the shroud or the frame and the optical filter are formed of the same light filtering material.

In at least one example, the optical filter is formed from silicon. The optical filter may be one or both of doped with a metallic coating or etched in a particular pattern to filter the UV light at a predetermined wavelength.

In at least one example, the coupling interface is common to the adaptable module and another adapter module that differs from the adaptable module.

Certain examples of the present disclosure provide a method including coupling an optical filter to a shroud or frame, wherein the optical filter is configured to filter ultraviolet (UV) light emitted by a UV lamp; and providing an adapter module by said coupling, wherein the adapter module is configured to removably couple to a sanitizing head having the UV lamp at a coupling interface.

Certain examples of the present disclosure provide an adaptable sanitizing system including a sanitizing head including an ultraviolet (UV) lamp configured to emit UV light; and an adapter module that removably couples to the sanitizing head, as described herein.

Certain examples of the present disclosure provide an adapter module configured to removably couple to a sanitizing head having an ultraviolet (UV) lamp that is configured to emit UV light. The adapter module includes a shroud that is configured to removably couple to the sanitizing head at a coupling interface, and a fluid passage in fluid communication with an inlet and an outlet within the shroud. The fluid passage is configured to receive fluid through the inlet and pass the fluid out of the outlet. The UV lamp is configured to emit the UV light into the fluid passage as the fluid passes through the fluid passage between the inlet and the outlet.

In at least one example, the fluid passage is defined by internal surfaces of the shroud. The internal surfaces of the shroud may be reflective.

In at least one example, the fluid is a gas. For example, the gas is air.

In at least one example, the fluid is a liquid. For example, the liquid is water.

In at least one example, a particulate filter is disposed within the fluid passage. In at least one example, an ozone filter is disposed within the fluid passage.

In at least one example, one or more tubes are within the shroud. The one or more tubes define the fluid passage. For example, the one or more tubes include a plurality of straight segments coupled to one or more bends. As an example, the one or more tubes are formed of glass.

In at least one example, a blower is disposed within the shroud.

In at least one example, the outlet connects to a tube that connects to a breathing mask.

In at least one example, one or both of a valve or a pump is proximate to the inlet. One or both of the valve or the pump are configured to control flow of liquid through the fluid passage.

Certain examples of the present disclosure provide a method including providing a fluid passage in fluid communication with an inlet and an outlet within a shroud of an adapter module, wherein fluid passes into the fluid passage through the inlet and passes out of the outlet, and wherein ultraviolet (UV) light is emitted into the fluid passage as the fluid passes through the fluid passage between the inlet and the outlet.

### BRIEF DESCRIPTION OF THE DRAWINGS

Figure 1 illustrates a schematic block diagram of an adaptable sanitizing system.
Figure 2 illustrates a flow chart of an adaptable sanitizing method.
Figure 3 illustrates a simplified view of a coupling interface between a sanitizing head and an adapter module.
Figure 4 illustrates a simplified view of a coupling interface between a sanitizing head and an adapter module.
Figure 5 illustrates a simplified view of a coupling interface between a sanitizing head and an adapter module.
Figure 6 illustrates a simplified view of a coupling interface between a sanitizing head and an adapter module.
Figure 7 illustrates a simplified view of a coupling interface between a sanitizing head and an adapter module.
Figure 8 illustrates a simplified view of a coupling interface between a sanitizing head and an adapter module.
Figure 9 illustrates a perspective bottom view of an adaptable sanitizing system.
Figure 10 illustrates a cross-sectional view of an adapter module through line 10-10 of Figure 9.
Figure 11 illustrates a perspective bottom view of an adaptable sanitizing system having an adapter module separated from a sanitizing head.
Figure 12 illustrates a schematic block diagram of an adapter module.
Figure 13 illustrates a perspective view of a sanitizing system having an adapter module uncoupled from a sanitizing head.
Figure 14 illustrates a perspective view of the sanitizing system of Figure 13 having the adapter module coupled to the sanitizing head.
Figure 15 illustrates a perspective top view of an adapter module.
Figure 16 illustrates a top view of a fluid passage.
Figure 17 illustrates an internal view of a sanitizing system.
Figure 18 illustrate an internal view of a sanitizing system.
Figure 19 illustrates a perspective view of a sanitizing system coupled to a breathing mask.
Figure 20 illustrates a side view of a sanitizing system.
Figure 21 illustrates a side view of a sanitizing system.
Figure 22 illustrates a side view of a sanitizing system.
Figure 23 illustrates a perspective view of a portable sanitizing system worn by an individual.
Figure 24 illustrates a perspective lateral top view of a wand assembly.
Figure 25 illustrates a perspective rear view of the wand assembly of Figure 24.
Figure 26 illustrates a perspective lateral view of the wand assembly of Figure 24.
Figure 27 illustrates a perspective view of the portable sanitizing system in a compact deployed position.
Figure 28 illustrates a perspective view of the portable sanitizing system having a sanitizing head in an extended position.
Figure 29 illustrates a perspective view of the portable sanitizing system having the sanitizing head in an extended position and a handle in an extended position.
Figure 30 illustrates a perspective view of the portable sanitizing system having the sanitizing head rotated in relation to the handle.
Figure 31 illustrates a perspective end view of a UV lamp and a reflector of the sanitizing head.
Figure 32 illustrates a perspective end view of a UV lamp and a reflector of the sanitizing head.
Figure 33 illustrates a perspective end view of a UV lamp and a reflector of the sanitizing head.
Figure 34 illustrates a perspective top view of the sanitizing head.
Figure 35 illustrates a perspective bottom view of the sanitizing head.
Figure 36 illustrates an axial cross-sectional view of the sanitizing head through line 36-36 of Figure 34.
Figure 37 illustrates a perspective end view of the UV lamp secured to a mounting bracket.
Figure 38 illustrates a perspective exploded view of a backpack assembly.
Figure 39 illustrates a perspective front view of a harness coupled to a backpack assembly.
Figure 40 illustrates an ultraviolet light spectrum.
Figure 41 illustrates a perspective view of a portable sanitizing system.
Figure 42 illustrates a perspective view of the portable sanitizing system having a case assembly in an open position.
Figure 43 illustrates a perspective view of the portable sanitizing system having the case assembly in the open position.
Figure 44 illustrates a perspective view of the portable sanitizing system having the case assembly in the open position.
Figure 45 illustrates a perspective lateral view of the wand assembly.
Figure 46 illustrates a perspective bottom view of the wand assembly of Figure 45.
Figure 47 illustrates a perspective front view of an aircraft.

### DETAILED DESCRIPTION OF THE DISCLOSURE

The foregoing summary, as well as the following detailed description of certain examples will be better understood when read in conjunction with the appended drawings. As used herein, an element or step recited in the singular and preceded by the word "a" or "an" should be understood as not necessarily excluding the plural of the elements or steps. Further, references to "one example" are not intended to be interpreted as excluding the existence of additional examples that also incorporate the recited features. Moreover, unless explicitly stated to the contrary, examples "comprising" or "having" an element or a plurality of elements having a particular condition can include additional elements not having that condition.

As described herein, examples of the present disclosure provide an adaptable sanitizing system including one or more adapter modules that are configured to removably couple to a sanitizing head having an ultraviolet (UV) lamp, which is configured to emit UV light. The adapter modules are configured to provide functionality in relation to the UV light emitted by the UV lamp. For example, the adapter modules are configured to filter UV light. The adapter modules can be used to disinfect one or more of the following: skin or wounds of individuals, surfaces of objects (such as tools and equipment), fluid, such as air or water, and/or the like.

The phrase or term "adapter module" is intended to mean a device or assembly or the like that is attachable and/or removable to a sanitizing head, for example, and assist and/or compliment a UV disinfection or sanitizing process.

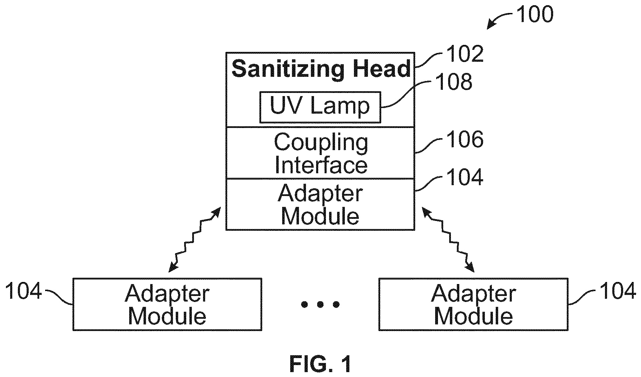

Figure 1 illustrates a schematic block diagram of an adaptable sanitizing system 100, according to an example of the present disclosure. The sanitizing system 100 includes a sanitizing head 102 and a plurality of adapter modules 104 that are configured to removably attach to the sanitizing head 102 at a coupling interface 106.

The sanitizing head 102 includes an ultraviolet (UV) lamp 108 that is configured to emit UV light. The sanitizing head 102 is configured for use without any of the adapter modules 104 secured thereto. For example, the sanitizing head 102 can be uncoupled from the adapter modules 104 and emit UV light onto a surface to disinfect the surface. In at least one example, the sanitizing head 102 is part of a portable, mobile platform. For example, the sanitizing head 102 is part of a wand assembly, as described herein. The wand assembly can be coupled to a backpack assembly, a case assembly, a cart assembly, or the like. As another example, the wand assembly can be a standalone unit that is not coupled to another assembly, such as a backpack assembly.

Each adapter module 104 is configured to removably secure (that is, quickly attach to, and detach from) the sanitizing head 102 at the coupling interface 106. As examples, the coupling interface 106 can include a track(s), a latch(es), a detent(s), an interference fit, a spring-biased coupling, an integral or separate fastener(s), and/or the like. In at least one example, the coupling interface 106 is common to all of the adapter modules 104. That is, the coupling interface 106 may be the same for all of the adapter modules 104.

The adapter modules 104 are configured to provide a functionality in relation to the UV light emitted from the UV lamp 108. Each of the adapter modules 104 can provide a different functionality from the others. Examples of the functionalities include optical filtering, optical wavelength converting, fluid sanitizing, object sanitizing, and/or the like.

As an example, at least one of the adapter modules 104 is an optical filter that is configured to optically filter the UV light so as to ensure that certain undesired wavelengths of the UV light are not emitted through the first adapter module 104. In this manner, the first adapter module 104 ensures that only UV light at a desired wavelength is emitted from the UV lamp 108 and through the first adapter module 104. As an example, the first adapter module 104 includes an optical filter, such as a low pass optical filter that filters out light above 230 nanometers (nm).

As another example, at least one of the adapter modules 104 is an optical converter that is configured to convert UV light emitted from the UV lamp 108 at a first wavelength to a second wavelength. For example, the adapter module 104 is configured to up-convert or down-convert the UV light emitted from the UV lamp 108.

As another example, at least one of the adapter modules 104 is a fluid sanitizer that is configured to disinfect a fluid stream passing therethrough via the UV light emitted from the UV lamp 108. For example, the adapter module 104 is configured to allow a gas (such as air) or a liquid (such as water) to pass through an internal channel. The UV light emitted from the UV lamp 108 disinfects the fluid as it passes through the internal channel.

As another example, at least one of the adapter module 104 is an object sanitizer that is configured to disinfect an object (such as a utensil, tool, ball, and/or the like) disposed within a chamber therein. In at least one example, the chamber may be configured to allow a portion of human anatomy, such as a hand, to be disposed therein. The UV light emitted by the UV lamp is configured to disinfect the portion of human anatomy.

Each of the adapter modules 104 can be configured for a different, unique functionality, such as optical filtration, optical wavelength conversion, fluid sanitation, object sanitation, and/or the like. For example, a first adapter module 104 includes an optical filter, a second adapter module 104 includes a wavelength converter, a third adapter module 104 includes a fluid sanitizer, a fourth adapter module 104 includes an object sanitizer, and/or the like. Each of the adapter module 104 is interchangeable and is configured to removably attach, secure, or otherwise coupled to the sanitizing head 102 at the coupling interface 106. In this manner, the sanitizing system 100 can be adapted to perform various different functions via the different adapter modules 104. The sanitizing system 100 can include more or less adapter modules 104 than shown.

As descried herein, the adaptable sanitizing system 100 includes the sanitizing head 102 including the UV lamp 108 configured to emit UV light. One or more adapter modules 104 are configured to removably couple to the sanitizing head 102 at the coupling interface 106. The adapter modules 104 are configured to provide a functionality (for example, optical filtering, optical wavelength converting, fluid sanitizing/disinfecting, object disinfecting, and/or the like) in relation to the UV light emitted by the UV lamp 108. For example, a first adapter module 104 is configured to perform a first unique functionality (such as optical filtering), and a second adapter module 104 configured to perform a second unique functionality (such as fluid sanitizing/disinfecting) that differs from the first unique functionality.

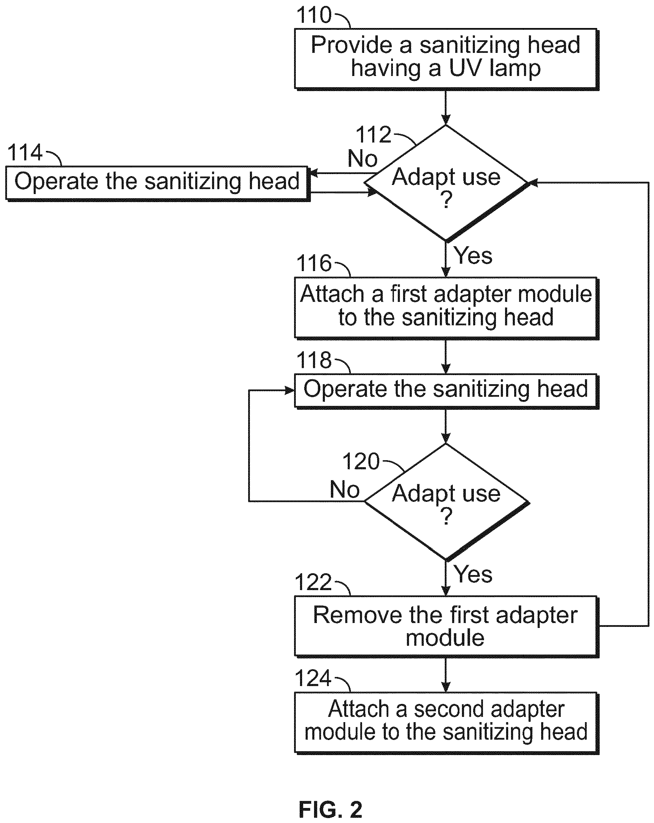

Figure 2 illustrates a flow chart of an adaptable sanitizing method, according to an example of the present disclosure. Referring to Figures 1 and 2, the method begins at 110, at which the sanitizing head 102 having the UV lamp 108 is provided. The sanitizing head 102 does not include any of the adapter modules 104. The sanitizing head 102 is configured to emit the UV light at a particular wavelength, such as within the far UV spectrum (for example, between 220-230 nm), the UVC spectrum (such as between 230-280 nm), and/or the like.

At 112, it is determined if use of the sanitizing head 102 is to be adapted (for example, changed). That is, it is determined if the particular use for which the sanitizing head 110 is configured is to be changed. For example, the sanitizing head 102 can be configured to sanitize a surface of an object at a particular wavelength, such as 222 nm, 254 nm, or the like. The method may then return to 112. If the use is not to be adapted at 112, the method proceeds to 114, at which the sanitizing head 102 is operated without any adapter module 104 coupled thereto.

If, however, it is determined that the use of sanitizing head 102 is to be adapted at 112, the method proceeds to 116, at which a first adapter module 104 (configured for a desired functionality) is attached to the sanitizing head 102. Next, the sanitizing head 102 is operated at 118 to achieve the desired functionality via the first adapter module 104.

At 120, it is determined if the use of the sanitizing head 102 coupled to the first adapter module 104 is to be adapted (for example, changed). If not, the method returns to 118.

If, however, the use of the sanitizing head 102 coupled to the first adapter module 104 is to be adapted, the method proceeds to 122, at which the first adapter module 104 is removed from the sanitizing head 102. The method may then return to 112. Optionally, the method may proceed to 124, at which a second adapter module 104, which is configured for a functionality that differs from the first adapter module 104, is attached to the sanitizing head 102. The method then repeats in a similar fashion.

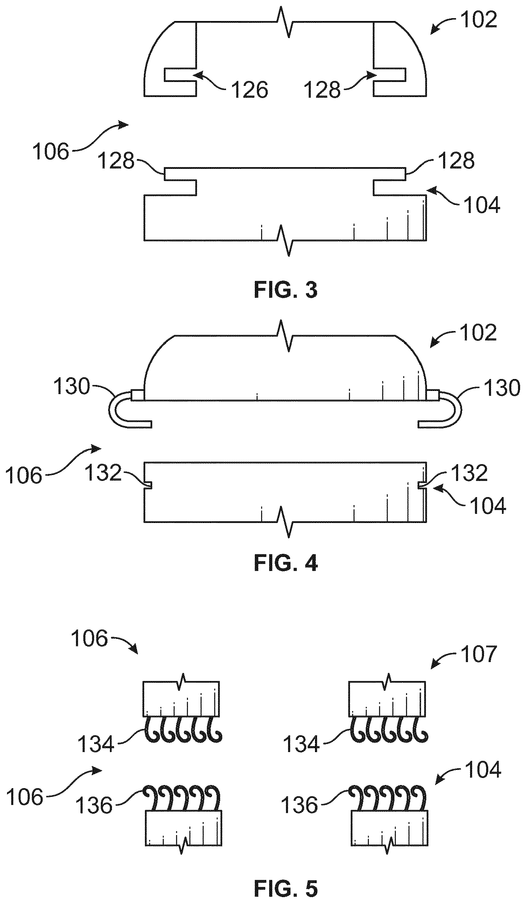

Figure 3 illustrates a simplified view of the coupling interface 106 between the sanitizing head 102 and the adapter module 104, according to an example of the present disclosure. For example, the coupling interface 106 includes one or more tracks 126 within a housing, body, or shroud of the sanitizing head 102 into which one or more reciprocal members 128 (such as ridges, tabs, ledges, fins, and/or the like) of the adapter module 104 are configured to slide. Optionally, the sanitizing head 102 includes the reciprocal members 128, and the adapter module 104 includes the track(s) 126. In at least one example, the coupling interface 106 is common to all of the adapter modules 104, such as shown in Figure 1.

Figure 4 illustrates a simplified view of the coupling interface 106 between the sanitizing head 102 and the adapter module 104, according to an example of the present disclosure. For example, the coupling interface 106 includes one or more latches 130 secured to a housing, body, or shroud of the sanitizing head 102. The latches 130 are configured to secure into and release from reciprocal members 132 (such as openings, protuberances, or the like) of the adapter module 104. Optionally, the sanitizing head 102 includes the reciprocal members 132, and the adapter module 104 includes the latch(es) 130. In at least one example, the coupling interface 106 is common to all of the adapter modules 104, such as shown in Figure 1.

Figure 5 illustrates a simplified view of the coupling interface 106 between the sanitizing head 102 and the adapter module 104, according to an example of the present disclosure. For example, the coupling interface 106 includes hook and loop fasteners 134 (for example, Velcro) secured to a housing, body, or shroud of the sanitizing head 102. The hook and loop fasteners 134 are configured to secure to and release from reciprocal hook and loop fasteners 136. For example, the hook and loop fasteners 134 are secured to exposed lower surfaces of the sanitizing head 102, and the hook and loop fasteners 136 are secured to upper surfaces of the adapter module 104. In at least one example, the coupling interface 106 is common to all of the adapter modules 104, such as shown in Figure 1.

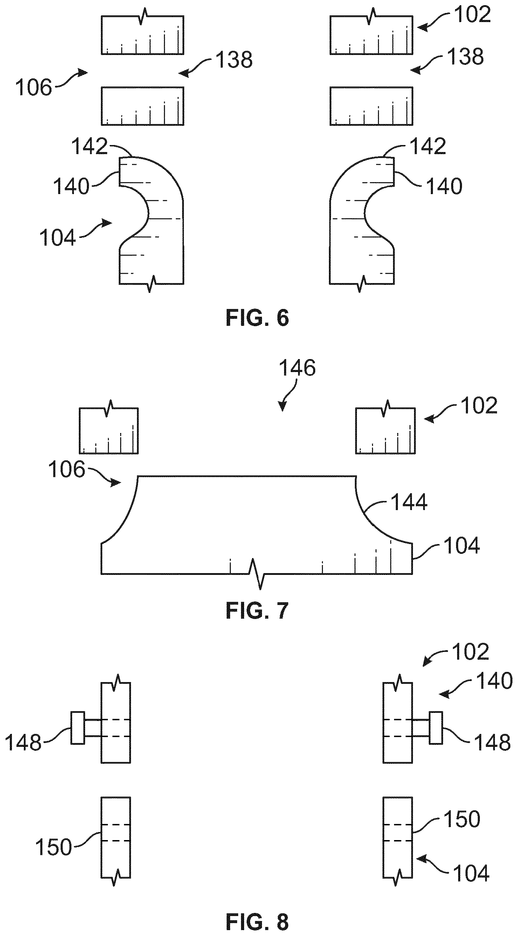

Figure 6 illustrates a simplified view of the coupling interface 106 between the sanitizing head 102 and the adapter module 104, according to an example of the present disclosure. For example, the coupling interface 106 includes one or more through-holes 138 secured to a housing, body, or shroud of the sanitizing head 102. One or more deflectable spring arms 140 of the adapter module 104 have distal ends 142 that are configured to deflect into (for example, snap into) the through-holes 138. The latches 130 are configured to secure into and release from reciprocal members 132 (such as openings, protuberances, or the like) of the adapter module 104. Optionally, the sanitizing head 102 includes the deflectable spring arms 140 and the adapter module 104 includes the through-holes 138. In at least one example, the coupling interface 106 is common to all of the adapter modules 104, such as shown in Figure 1.

Figure 7 illustrates a simplified view of the coupling interface 106 between the sanitizing head 102 and the adapter module 104, according to an example of the present disclosure. For example, the coupling interface 106 includes a surface 144 that is configured to fit into an opening 146 of the sanitizing head 102 through an interference fit and/or plug and socket connection. Optionally, the sanitizing head 102 includes the surface 144, and the adapter module 104 includes the opening 146. In at least one example, the coupling interface 106 is common to all of the adapter modules 104, such as shown in Figure 1.

Figure 8 illustrates a simplified view of the coupling interface 106 between the sanitizing head 102 and the adapter module 104, according to an example of the present disclosure. For example, the coupling interface 106 includes one or more integral fasteners 148 (such as quarter turn, half turn, or the like bayonet-type fasteners) that are configured to mate with reciprocal cavities 150 formed in the adapter module 104. Optionally, the sanitizing head 102 includes the cavities 150, and the adapter module 104 includes the fasteners 148. In at least one example, the coupling interface 106 is common to all of the adapter modules 104, such as shown in Figure 1.

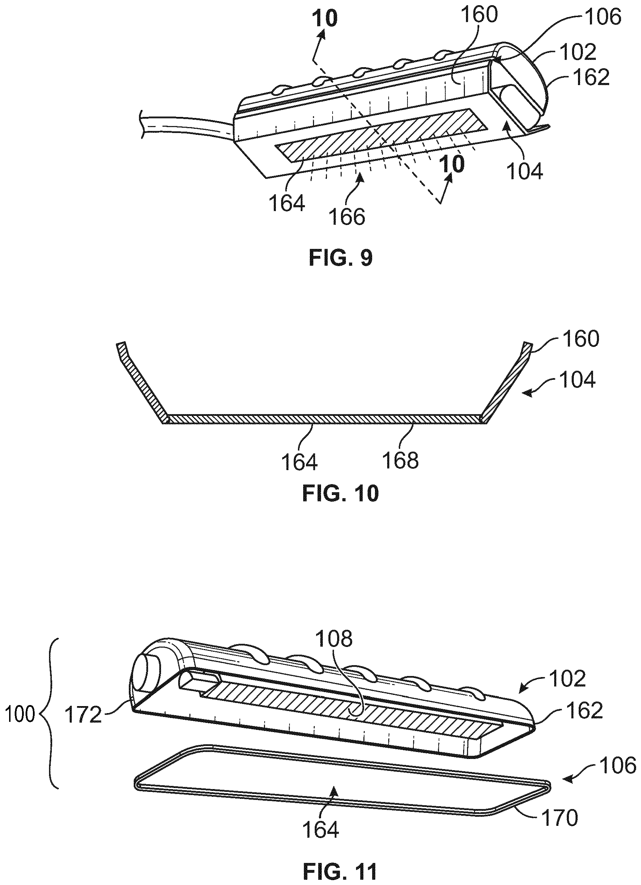

Figure 9 illustrates a perspective bottom view of the adaptable sanitizing system 100, according to an example of the present disclosure. The adaptable sanitizing system 100 includes the sanitizing head 102, and the adapter module 104, which is removably coupled to the sanitizing head 102, as described herein. In at least one example, the adapter module 104 includes a shroud 160 that removably secures to a shroud 162 of the sanitizing head 102 at the coupling interface 106. The shroud 160 retains an optical filter 164. Referring to Figures 1 and 9, UV light emitted by the UV lamp 108 is emitted toward the optical filter 164, which filters light at predetermined wavelengths from the emitted UV lamp 108, thereby ensuring that filtered UV light 166 passes out of the optical filter 164.

Referring to Figures 1 and 9, the adapter module 104 is distinct from the sanitizing head 102. That is, the sanitizing head 102 does not include the adapter module 104, or vice versa. Rather, the adapter module 104 is configured to removably couple to (for example, connect to, and disconnect from) the sanitizing head 102. The sanitizing head 102 is operative (such as to emit UV light) whether or not the adapter module 104 is secured to the sanitizing head 102.

As an example, the UV lamp 108 is configured to emit UV light at a particular wavelength, such as within the far UV spectrum (for example, 222 nm). However, the UV light emitted by the UV lamp 108 may still include light at longer wavelengths, such as beyond 230 nm. Accordingly, in at least one example, the optical filter 164 is a 230 nm low pass filter that filters light above 230 nm, thereby ensuring that light having a wavelength shorter than 230 nm passes through and out of the optical filter 164. As an example, the optical filter 164 filters the UV light emitted by the UV lamp 108, thereby providing the UV light 166 that passes through and out of the optical filter 164 at a wavelength of 222 nm, which neutralizes (such as kills) microbes (for example, viruses and bacteria), while posing no risk to humans.

As can be appreciated, however, the optical filter 164 attenuates or otherwise causes a loss of power from the UV light as it passes therethrough. As such, if more power is desired, and if there is no risk to human exposure, the adapter module 104 can be removed from the sanitizing head 102, which may then emit the UV light at a higher, unfiltered power. The adapter module 104 can be selectively connected to, and removed from, the sanitizing head 102 as desired.

As described, in at least one example, the sanitizing system 100 includes the adapter module 104 having the optical filter 164. The adapter module 104 can be selectively coupled to an uncoupled from the sanitizing head 102. The sanitizing system 100 can be used without the adapter module 104 to emit UV light onto surfaces, when there is little to no risk of human exposure. If, however, the potential for human exposure exists, the adapter module 104 can be coupled to the sanitizing head 102 so that the optical filter 164 efficiently filters undesired wavelengths of UV light.

Figure 10 illustrates a cross-sectional view of the adapter module 104 through line 10-10 of Figure 9. In at least one example, the optical filter 164 is a panel 168 that secures to the shroud 160. In at least one other example, the entire adapter module 104 can be or otherwise include a homogenous optical filter 164. For example, the adapter module 104 can be formed of the material of the optical filter 164. In at least one example, the shroud 160 and the optical filter 164 are formed of the same, light filtering material.

In at least one example, the optical filter 164 is formed of silicon. The optical filter 164 can be doped with a metallic coating and/or etched in a particular pattern to filter UV light at a predetermined wavelength. As noted, the optical filter 164 can be a 230 nm low pass filter that is configured to filter wavelengths above 230 nm from the UV light emitted by the UV lamp 108. Optionally, the optical filter 164 can be configured to filter wavelengths higher or lower than 230 nm. For example, the optical filter 164 can be configured to filter wavelengths other than 254 nm from the UV light emitted by the UV lamp 108.

Figure 11 illustrates a perspective bottom view of an adaptable sanitizing system 100 having the adapter module 104 separated from the sanitizing head 102, according to an example of the present disclosure. In at least one example, the adapter module 104 includes a frame 170 that retains the optical filter 164 therebetween. The frame 170 is configured to removably couple to an exposed lower perimeter 172 of the shroud 162 of the sanitizing head 102 via a coupling interface 106, such as any of those described herein.

Referring to Figures 1 and 9-11, an adapter module 104 is configured to removably couple to the sanitizing head 102 having the UV lamp 108 that is configured to emit UV light. The adapter module includes the shroud 160 or the frame 170 that is configured to removably couple to the sanitizing head 102 at the coupling interface 106. The optical filter 164 is coupled to the shroud 160 or the frame 170. The optical filter 164 is configured to filter the UV light emitted by the UV lamp 108.

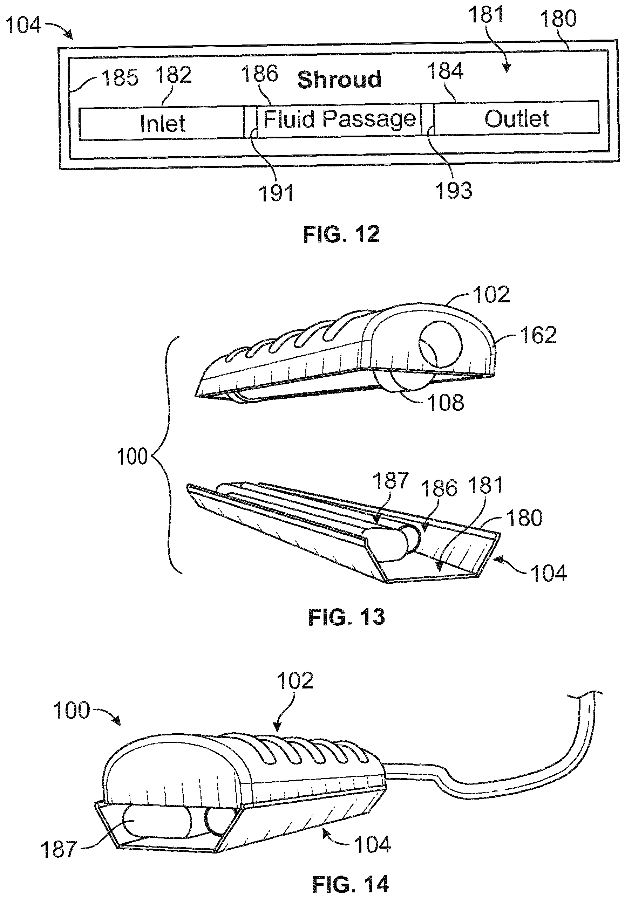

Figure 12 illustrates a schematic block diagram of an adapter module 104, according to an example of the present disclosure. As shown, the adapter module 104 includes a shroud 180. The shroud 180 includes an inlet 182 and an outlet 184 in fluid communication with a fluid passage 186 within the shroud 180. The fluid passage 186 can be defined by internal surfaces 185 of the shroud 180. In at least one example, the internal surfaces 185 are reflective. For example, the internal surfaces 185 are coated and/or covered with mirrors or other such reflectors (such as Teflon, Porex, polished aluminum, and or the like).

In operation, fluid (such as a gas or liquid) enters the shroud through the inlet 152 and passes through the fluid passage 186. Referring to Figures 1 and 12, UV light emitted by the UV lamp 108 is emitted into the fluid passage 186, thereby disinfecting the fluid. The disinfected fluid then passes out of the outlet 184.

The reflective surfaces 185 reflect the UV light back into the fluid passage 186. Further, the sanitizing head 102 can also include internal reflective surfaces, thereby ensuring that the emitted UV light continually reflects into the fluid passage 186 (with no or reduced absorption), which increases the efficiency of the sanitizing/disinfecting operation. Alternatively, the adapter module 104 may not include reflective surfaces.

In at least one example, a particulate filter 191 is disposed within the fluid passage 186. For example, the particulate filter 191 is disposed at or proximate to the inlet 182. The particulate filter 191 removes particulates (such as dust, debris, or other impurities) from the fluid, whether gas or liquid. Particulates could otherwise cause shadowing in relation to the UV light. As such, the particulate filter 191 increases the effectiveness and efficiency of the sanitation process. Alternatively, the adapter module 104 does not include the particulate filter.

In at least one example, an ozone filter 193 is disposed within the fluid passage 186. For example, the ozone filter 193 is disposed at or proximate to the outlet 184. The ozone filter 193 removes ozone (such as may be generated by the UV light interacting with the fluid stream) from the fluid stream. Alternatively, the adapter module 104 does not include the ozone filter.

Referring to Figures 1 and 12, an adapter module 104 is configured to removably couple to the sanitizing head 102 having the UV lamp 108 that is configured to emit UV light. The adapter module 104 includes the shroud 180 that is configured to removably couple to the sanitizing head 102 at the coupling interface 106. The fluid passage 186 in fluid communication with the inlet 182 and the outlet 184 is within the shroud 180 (such as within an internal chamber 181 defined by the shroud 180. Fluid (such as a gas or liquid) passes into the fluid passage 186 through the inlet 182 and passes out of the outlet 184. The UV light is emitted by the UV lamp 108 into the fluid passage 186 as the fluid passes through the fluid passage 186 between the inlet 182 and the outlet 184.

Figure 13 illustrates a perspective view of the sanitizing system 100 having the adapter module 104 uncoupled from the sanitizing head 102, according to an example of the present disclosure. Figure 14 illustrates a perspective view of the sanitizing system 100 of Figure 13 having the adapter module 104 coupled to the sanitizing head 102. Referring to Figures 12-14, the shroud 180 of the adapter module 104 can define the fluid passage 186. In at least one other example, one or more tubes 187 (such as formed of glass) can define the fluid passage 186.

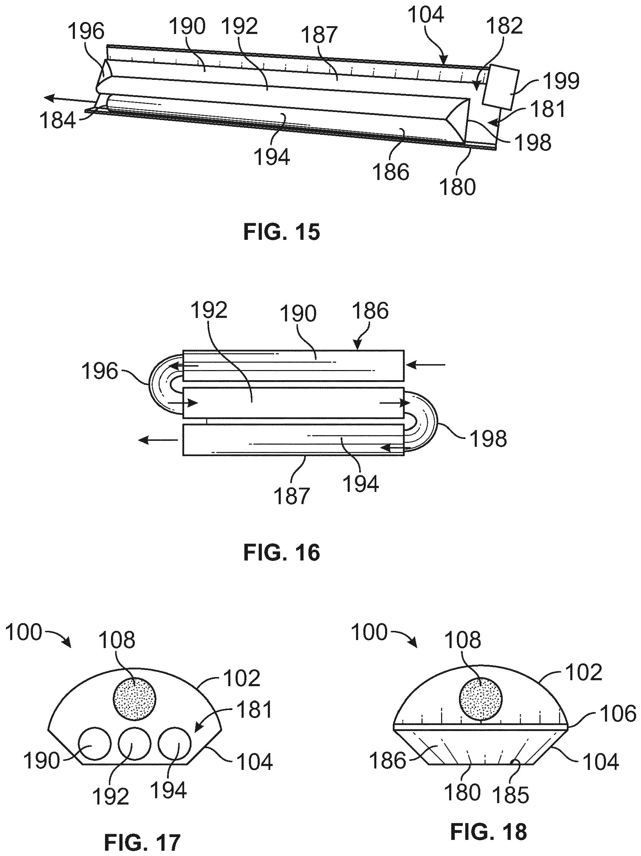

Figure 15 illustrates a perspective top view of the adapter module 104, according to an example of the present disclosure. Figure 16 illustrates a top view of the fluid passage 186, according to an example of the present disclosure. Figure 17 illustrates an internal view of the sanitizing system 100. Referring to Figures 15-17, as shown, the adapter module 104 includes a tube 187 having a plurality of straight segments 190, 192, and 194 connected by bends 196 and 198, thereby providing a long, circuitous path for fluid to travel within the adapter module 104. The fluid can be a gas (such as air), or liquid (such as water).

In at least one example, a blower 199, such as a fan, is disposed within the adapter module 104 (such as within and/or on the shroud 180). The blower 199 can be disposed at or proximate to (such as within 3 inches) the inlet 182, for example. Optionally, the blower 199 can be disposed within the tube 187. The blower 199 draws air from outside the adapter module 104 into the fluid passage 186.

In at least one example, the tube 187 is formed of a transparent material, such as glass. It has been found that a glass tube 187 absorbs little to no UV light, thereby ensuring that the UV light emitted by the UV lamp 108 effectively and efficiently disinfects the fluid flowing through the fluid passage 186, as defined by the tube 187. The multiple segments 190, 192, and 194, and bends 196 and 198 provided a relatively long, circuitous path for the fluid to travel, which extends the time the fluid is within the fluid passage 186. The extended length of time of the fluid within the fluid passage 186 ensures a longer period of time of exposure to the UV light, which further increasing the sanitizing efficiency.

Optionally, the tube 187 may include more or less segments and bends than shown. For example, the tube 187 may include a single straight segment with no bends. As another example, the tube 187 may include four or more straight segments and three or more bends. As another example, multiple disconnected tubes 187 may be used.

Figure 18 illustrate an internal view of the sanitizing system 100, according to an example of the present disclosure. In this example, the adapter module 104 does not include a tube. Instead, the fluid passage 186 is defined by internal surfaces 185 (whether or not reflective) of the shroud 180.

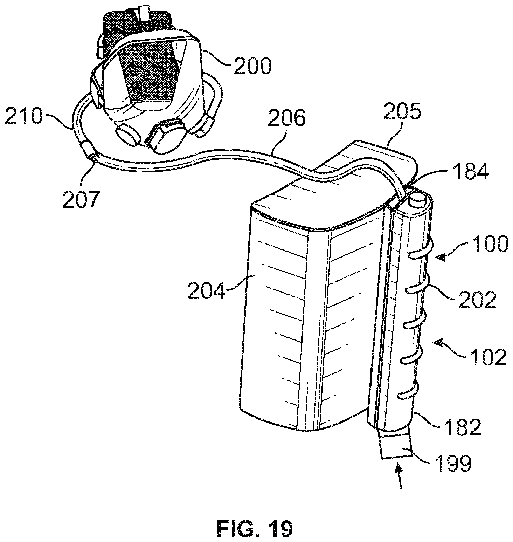

Figure 19 illustrates a perspective view of the sanitizing system 100 coupled to a breathing mask 200, according to an example of the present disclosure. The sanitizing head 102 can be part of a wand assembly 202 that couples to a backpack assembly 204. The adapter module 104 is configured to sanitize fluid, such as described with respect to Figures 12-19. A first end 205 of a hose or tube 206 connects to the outlet 184. A second end 207 of the tube 206 connects to an inlet 210 of the breathing mask 200. As such, the sanitizing system 100 is configured, by way of the adapter module 104, to sanitize air, and deliver the clean, sanitized air to the breathing mask 200, which can be worn by an individual. The sanitizing system 100 and the breathing mask 200 can be used by an individual in a setting that may be susceptible to airborne pathogens, such as within a pandemic or epidemic zone.

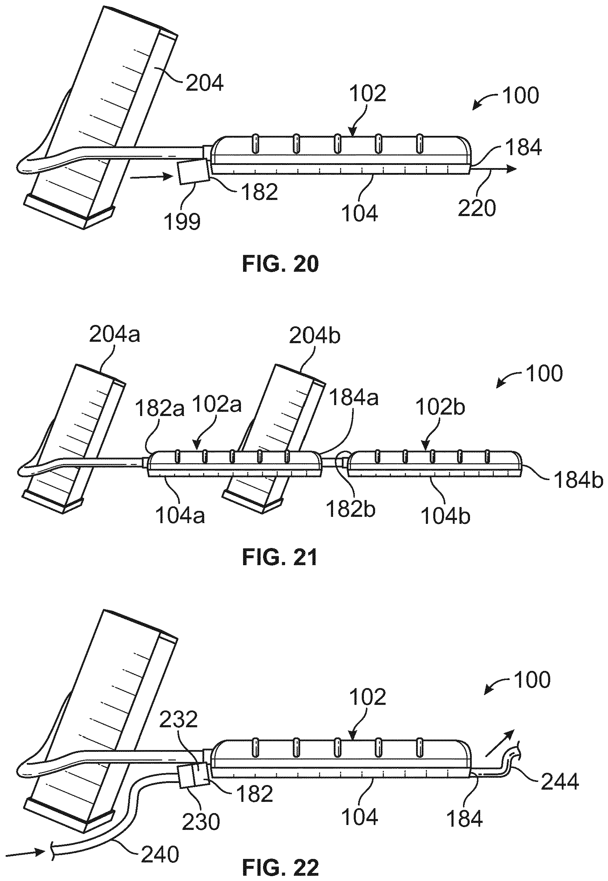

Figure 20 illustrates a side view of a sanitizing system 100, according to an example of the present disclosure. In this example, the sanitizing system 100 can be disposed at a location, such as within an internal cabin of a vehicle, a room, or the like. The blower 199 draws air into the adapter module 104. The air passes through the fluid passage 186, as described with respect to Figures 12-18. UV light emitted by the UV lamp 108 sanitizes the air as it passes through the fluid passage 186. The clean, sanitized air 220 is then passed out of the outlet 184 into the location.

Figure 21 illustrates a side view of a sanitizing system 100, according to an example of the present disclosure. In this example, an outlet 184a of a first adapter module 104a (coupled to a first sanitizing head 102a) is in fluid communication with an inlet 182b of a second adapter module 104b (coupled to a second sanitizing head 102b). In this manner, air that enters the inlet 182a of the first adapter module 104a is initially sanitized, and the initially sanitized air passes out of the outlet 184a into the inlet 182b of the second adapter module 104b, where it is further sanitized, and ultimately passes out of the outlet 184b into the location. As such, multiple adapter modules 104 can be linked together in series to provide highly sanitized air. The sanitizing system 100 can include more sanitizing heads and adapter modules than shown. The sanitizing head 102a may be coupled to a first backpack assembly 204a, and the sanitizing head 102b may be coupled to a second backpack assembly 204b.

Figure 22 illustrates a side view of a sanitizing system, 100 according to an example of the present disclosure. Referring to Figures 12-18 and 22, in at least one example, the fluid passage 186 can be configured to channel liquid, such as water. The adapter module 104 includes a valve 230 and pump 232 at or proximate to the inlet 182. A liquid inlet line 240 is connected to the valve 230, the pump 232, and/or the inlet 182. The valve 230 controls flow of the liquid into the fluid passage 186. The pump 232 moves the liquid through the fluid passage 186. The UV lamp 108 emits UV light into the fluid passage 186 as the liquid flows therethrough, thereby sanitizing the liquid as it flows through the fluid passage 186. Clean, sanitized liquid, such as may be suitable for drinking, flows out of the outlet 184, such as into an outlet hose 244. Optionally, the adapter module 104 may not include the valve 230 and/or the pump 232.

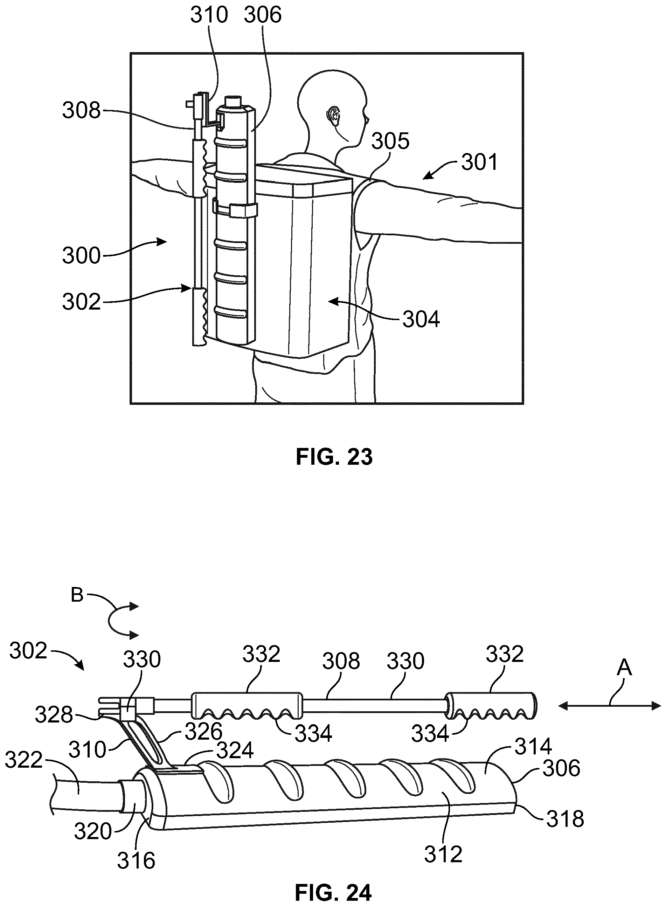

Figure 23 illustrates a perspective view of a portable sanitizing system 300 worn by an individual 301, according to an example of the present disclosure. The portable sanitizing system 300 includes a wand assembly 302 coupled to a backpack assembly 304 that is removably secured to the individual through a harness 305. The wand assembly 302 includes a sanitizing head 306 coupled to a handle 308. In at least one example, the sanitizing head 306 is moveably coupled to the handle 308 through a coupler 310.

The sanitizing head 306 is an example of the sanitizing head 102 shown and described with respect to Figures 1-22. Adapter modules 104 can be removably coupled to the sanitizing head 102, as described above.

In at least one other example, the portable sanitizing system 300 may not be worn by the individual 301. For example, the portable sanitizing system 300 may include a case assembly that is configured to be opened and closed. The case assembly may store the wand assembly 302 when not in use. The case assembly may be opened to allow the wand assembly 302 to be removed and operated.

As shown in Figure 23, the wand assembly 302 is in a stowed position. In the stowed position, the wand assembly 302 is removably secured to a portion of the backpack assembly 304, such as through one or more tracks, clips, latches, belts, ties, and/or the like.

In at least one other example, the wand assembly 302 is stored within a case assembly in a stowed position. For example, the wand assembly 302 in the stowed position is contained within a closed case assembly. The case assembly may be opened to allow the wand assembly 302 to be removed and deployed.

Figure 24 illustrates a perspective lateral top view of the wand assembly 302, according to an example of the present disclosure. The sanitizing head 306 couples to the handle 308 through the coupler 310. The sanitizing head 306 includes a shroud 312 having an outer cover 314 that extends from a proximal end 316 to a distal end 318. As described herein, the shroud 312 contains a UV lamp.

Optionally, the wand assembly 302 may include the sanitizing head 306 connected to a fixed handle. Further, the wand assembly 302 may be sized and shaped differently than shown.

A port 320 extends from the proximal end 316. The port 320 couples to a hose 322, which, in turn, couples to the backpack assembly 304 (shown in Figure 23). The hose 322 contains electrical cords, cables, wiring, or the like that couples a power source or supply (such as one or more batteries) within the backpack assembly 304 (shown in Figure 1) to a UV lamp 340 within the shroud 312. Optionally, the electrical cords, cables, wiring, or the like may be outside of the hose 322. In at least one example, the hose 322 also contains an air delivery line, such as an air tube) that fluidly couples an internal chamber of the shroud 312 to an air blower, vacuum generator, air filters, and/or the like within the backpack assembly 304.

The coupler 310 is secured to the outer cover 314 of the shroud 312, such as proximate to the proximal end 316. The coupler 310 may include a securing beam 324 secured to the outer cover 314, such as through one or more fasteners, adhesives, and/or the like. An extension beam 326 outwardly extends from the securing beam 324, thereby spacing the handle 308 from the shroud 312. A bearing assembly 328 extends from the extension beam 326 opposite from the securing beam 324. The bearing assembly 328 includes one or more bearings, tracks, and/or the like, which allow the handle 308 to linearly translate relative to the coupler 310 in the directions of arrows A, and/or pivot about a pivot axle in the directions of arc B. Optionally, the securing beam 324 may include a bearing assembly that allows the sanitizing head 306 to translate in the directions of arrows A, and/or rotate (for example, swivel) in the directions of arc B in addition to, or in place of, the handle 308 being coupled to the bearing assembly 328 (for example, the handle 308 may be fixed to the coupler 310).

In at least one other example, the wand assembly 302 does not include the coupler 310. Instead, the handle 308 may be fixed to the shroud 312, for example.

In at least one example, the handle 308 includes a rod, pole, beam, or the like 330, which may be longer than the shroud 312. Optionally, the rod 330 may be shorter than the shroud 312. One or more grips 332 are secured to the rod 330. The grips 332 are configured to be grasped and held by an individual. The grips 332 may include ergonomic tactile features 334.

Optionally, the wand assembly 302 can be sized and shaped differently than shown. For example, in at least one example, the handle 308 can be fixed in relation to the shroud 312. Further, the handle 308 may not be configured to move relative to itself and/or the shroud 312. For example, the handle 308 and the shroud 312 can be integrally molded and formed as a single unit.

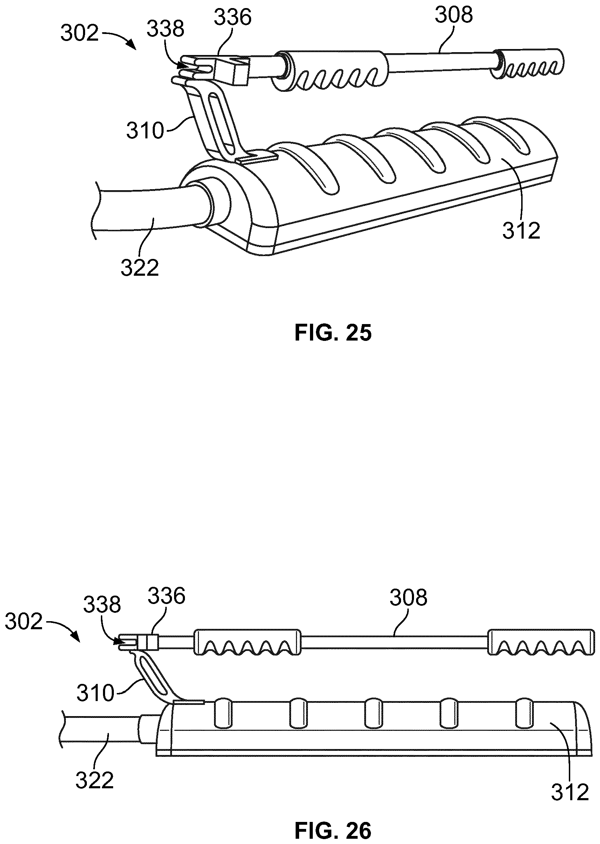

Figure 25 illustrates a perspective rear view of the wand assembly 302 of Figure 24. Figure 26 illustrates a perspective lateral view of the wand assembly 302 of Figure 24. Referring to Figures 25 and 26, the handle 308 may pivotally couple to the coupler 310 through a bearing 336 having a pivot axle 338 that pivotally couples the handle 308 to the coupler 310. The handle 308 may further be configured to linearly translate into and out of the bearing 336. For example, the handle 308 may be configured to telescope in and out. Optionally, or alternatively, in at least one example, the handle 308 may include a telescoping body that allows the handle 308 to outwardly extend and inwardly recede. In at least one other example, the handle 308 may not be configured to move, extend, retract, or the like relative to the shroud 312.

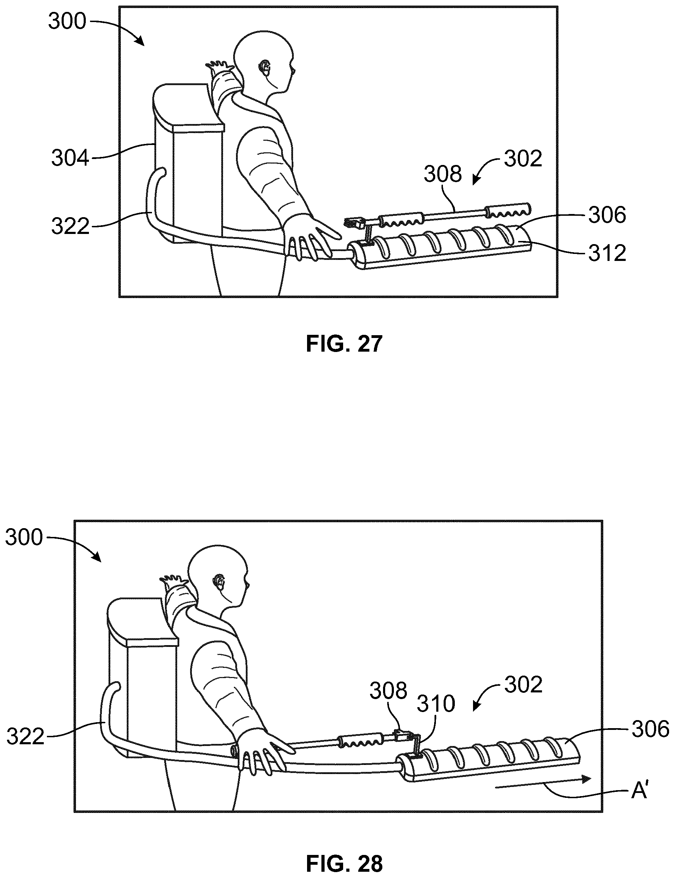

Figure 27 illustrates a perspective view of the portable sanitizing system 300 in a compact deployed position, according to an example of the present disclosure. The wand assembly 302 is removed from the backpack assembly 304 (as shown in Figure 23) into the compact deployed position, as shown in Figure 27. The hose 322 connects the wand assembly 302 to the backpack assembly 304. In the compact deployed position, the sanitizing head 306 is fully retracted in relation to the handle 308.

Figure 28 illustrates a perspective view of the portable sanitizing system 300 having the sanitizing head 306 in an extended position, according to an example of the present disclosure. In order to extend the sanitizing head 306 relative to the handle 308, the sanitizing head 306 is outwardly slid relative to the handle 308 in the direction of arrow A' (or the handle 308 is rearwardly slid relative to the sanitizing head 306). As noted, the sanitizing head 306 is able to linearly translate in the direction of arrow A' relative to the handle 308 via the coupler 310. The outward extension of the sanitizing head 306, as shown in Figure 28, allows for the portable sanitizing system 300 to easily reach distant areas. Alternatively, the sanitizing head 306 may not linearly translate relative to the handle 308.

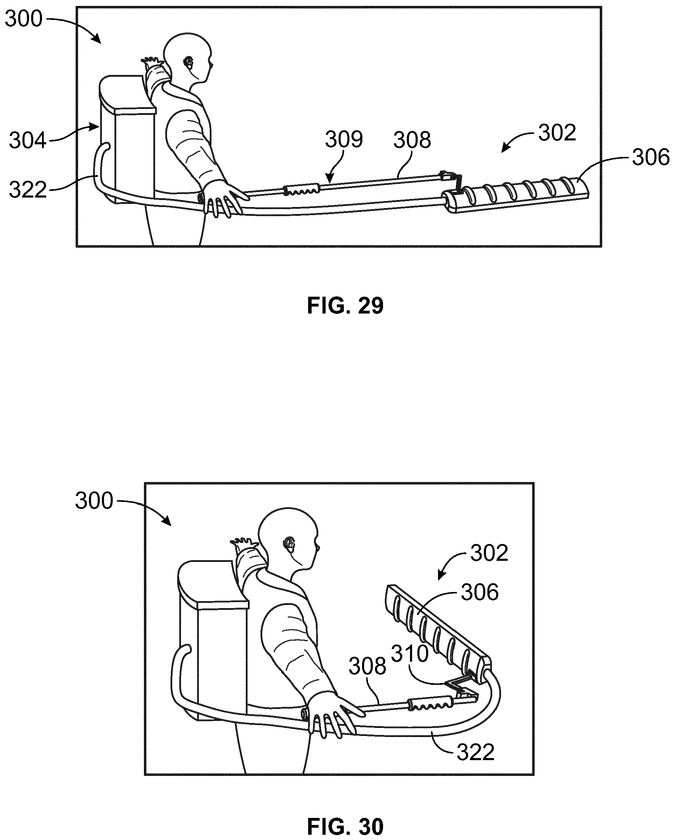

Figure 29 illustrates a perspective view of the portable sanitizing system 300 having the sanitizing head 306 in an extended position and the handle 308 in an extended position, according to an example of the present disclosure. To reach even further, the handle 308 may be configured to linearly translate, such as through a telescoping portion, to allow the sanitizing head 306 to reach further outwardly. Alternatively, the handle 308 may not be configured to extend and retract.

In at least one example, the handle 308 may include a lock 309. The lock 309 is configured to be selectively operated to secure the handle 308 into a desired extended (or retracted) position.

Figure 30 illustrates a perspective view of the portable sanitizing system 300 having the sanitizing head 306 rotated in relation to the handle 308, according to an example of the present disclosure. As noted, the sanitizing head 306 is configured to rotate relative to the handle 308 via the coupler 310. Rotating the sanitizing head 306 relative to the handle 308 allows the sanitizing head 306 to be moved to a desired position, and sweep or otherwise reach into areas that would otherwise be difficult to reach if the sanitizing head 306 was rigidly fixed to the handle 308. Alternatively, the sanitizing head 306 may not be rotatable relative to the handle 308.

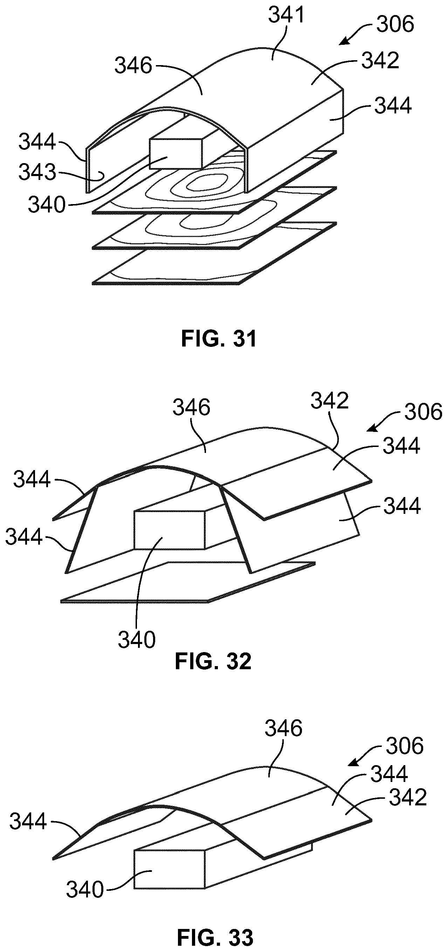

Figure 31 illustrates a perspective end view of a UV lamp 340 and a reflector 342 of the sanitizing head 306, according to an example of the present disclosure. Again, the sanitizing head 306 is an example of the sanitizing head 102, such as shown in Figure 1. Further, the UV lamp 340 is an example of the UV lamp 108, such as shown in Figure 1.

The UV lamp 340 and the reflector 342 are secured within the shroud 312 (shown in Figure 24, for example) of the sanitizing head 306. In at least one example, the reflector 342 is secured to an underside 341 of the shroud 312, such as through one or more adhesives. As another example, the reflector 342 is an integral part of the shroud 312. For example, the reflector 342 may be or otherwise provide the underside 341 of the shroud 312. The reflector 342 provides a reflective surface 343 (such as formed of Teflon, a mirrored surface, and/or the like) that is configured to outwardly reflect UV light emitted by the UV lamp 340. In at least one example, the shroud 312 may be or include a shell formed of fiberglass, and the reflector 342 may be formed of Teflon that provides a 98% reflectivity. In at least one example, the reflector 342 may be a multi-piece reflector.

The reflector 342 may extend along an entire length of the underside 341 of the shroud 312. Optionally, the reflector 342 may extend along less than an entire length of the underside 341 of the shroud 312.

The UV lamp 340 may extend along an entire length (or along substantially the entire length, such as between the ends 316 and 318). The UV lamp 340 is secured to the reflector 342 and/or the shroud 312 through one or more mounts, such as brackets, for example. The UV lamp 340 includes one or more UV light emitters, such as one more bulbs, light emitting elements (such as light emitting diodes), and/or the like. In at least one example, the UV lamp 340 is configured to emit UV light in the far UV spectrum, such as at a wavelength between 200 nm - 230 nm. In at least one example, the UV lamp 340 is configured to emit UV light having a wavelength of 222 nm. For example, the UV lamp 340 may be or include a 300 W bulb that is configured to emit UV light having a wavelength of 222 nm. Alternatively, the UV lamp 340 may be configured to emit UV light in other portions of the UV spectrum, such as the UVC spectrum.

As shown, the reflector 3142 includes flat, upright side walls 344 connected together through an upper curved wall 346. The upper curved wall 346 may be bowed outwardly away from the UV lamp 340. For example, the upper curved wall 346 may have a parabolic cross-section and/or profile.

It has been found that the straight, linear side walls 344 provide desired reflection and/or focusing of UV light emitted from the UV lamp 340 toward and onto a desired location. Alternatively, the side walls 344 may not be linear and flat.

Figure 32 illustrates a perspective end view of the UV lamp 340 and a reflector 342 of the sanitizing head, according to an example of the present disclosure. The reflector 342 shown in Figure 32 is similar to the reflector 342 shown in Figure 31, except that the side walls 344 may outwardly cant from the upper curved wall 346.

Figure 33 illustrates a perspective end view of the UV lamp 340 and the reflector 342 of the sanitizing head, according to an example of the present disclosure. In this example, the side walls 344 may be curved according to the curvature of the upper curved wall 346.

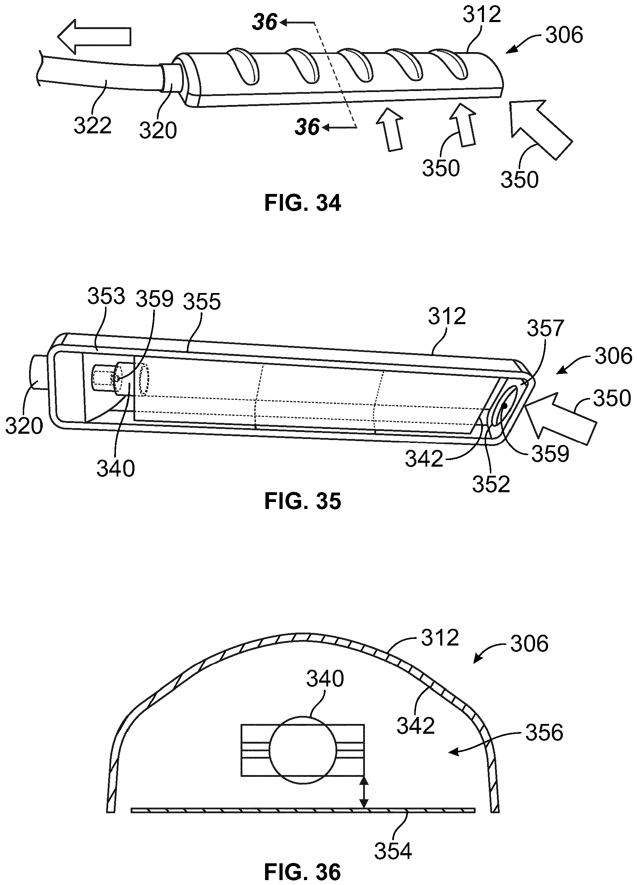

Figure 34 illustrates a perspective top view of the sanitizing head 306. Figure 35 illustrates a perspective bottom view of the sanitizing head 306. Figure 36 illustrates an axial cross-sectional view of the sanitizing head 306 through line 36-36 of Figure 34. Referring to Figures 34-36, air 350 is configured to be drawn into the sanitizing head 306 through one or more openings 352 (or simply an open chamber) of the shroud 312. The air 350 is drawn into the sanitizing head 306, such as via a vacuum generator within the backpack assembly 304 (shown in Figure 23). The air 350 is drawn into the shroud 312, and cools the UV lamp 340 as it passes over and around the UV lamp 340. The air 350 passes into the port 320 and into the hose 322, such as within an air tube within the hose 322. The air 350 not only cools the UV lamp 340, but also removes ozone, which may be generated by operation of the UV lamp 340, within the shroud 312. The air 350 may be drawn to an air filter, such as an activated carbon filter, within the backpack assembly 304.

In at least one example, the portable sanitizing system 300 may also include an alternative ozone mitigation system. As an example, the ozone mitigation system may be disposed in the shroud 312 or another portion of the system, and may include an inert gas bath, or a face inert gas system, such as in U.S. Patent No. 10,232,954.

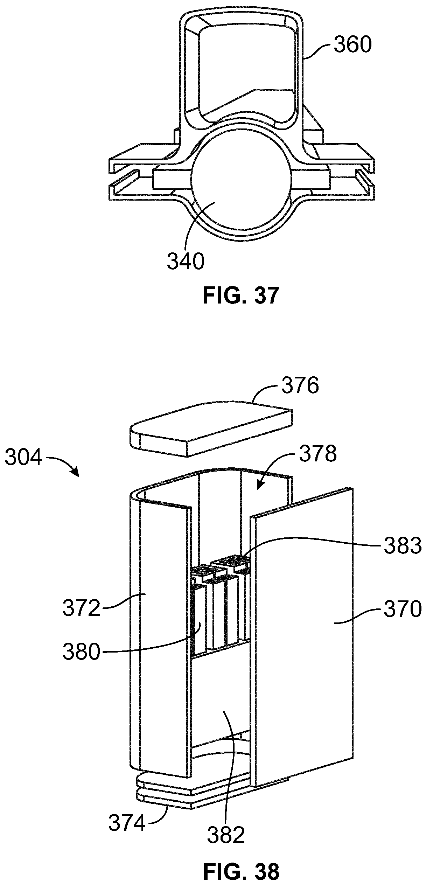

Referring to Figure 37, in particular, a bumper 353 may be secured to an exposed lower circumferential edge 355 of the shroud 312. The bumper 353 may be formed of a resilient material, such as rubber, another elastomeric material, open or closed cell foam, and/or the like. The bumper 353 protects the sanitizing head 306 from damage in case the sanitizing head 306 inadvertently contacts a surface. The bumper 353 also protects the surface from damage.

The openings 352 may be spaced around the lower surface of the shroud 312 such that they do not provide a direct view of the UV lamp 340. For example, the openings 352 may be positioned underneath portions that are spaced apart from the UV lamp 340.

Referring to Figure 36, in particular, the sanitizing head 306 may include a cover plate 354 below the UV lamp 340. The cover plate 354 may be formed of glass, for example, and may be configured to filter UV light emitted by the UV lamp 340. The UV lamp 340 may be secured within an interior chamber 356 defined between the reflector 342 and the cover plate 354. In at least one example, the cover plate 354 is or otherwise includes a far UV band pass filter. For example, the cover plate 354 may be a 222 nm band pass filter that filters UV light emitted by the UV lamp 340 to a 222 nm wavelength. As such, UV light that is emitted from the sanitizing head 306 may be emitted at a wavelength of 222 nm. The optical filter 164, shown and described with respect to Figures 9-11, may be a 222 nm band pass filter.

Referring to Figures 35 and 36, a rim 357 (such as a 0.020" thick Titanium rim) may connect the cover plate 354 to the shroud 312. The rim 357 may distribute impact loads therethrough and/or therearound.

In at least one example, ranging light emitting diodes (LEDs) 359 may be disposed proximate to ends of the UV lamp 340. The ranging LEDs 359 may be used to determine a desired range to a structure that is to be sanitized, for example. In at least one example, the ranging LEDs 359 may be disposed on or within the rim 357 and/or the cover plate 354. As another example, the sanitizing head 306 may be configured for range guidance, as disclosed in United States Provisional Application No. 63/027,869, which was filed May 20, 2020.

Figure 37 illustrates a perspective end view of the UV lamp 340 secured to a mounting bracket or clamp 360, according to an example of the present disclosure. Each end of the UV lamp 340 may be coupled to mounting bracket or clamp 360, which secures the UV lamp 340 to the shroud 312 (shown in Figures 34-36). A buffer, such as a thin (for example, 0.040") sheet of silicon may be disposed between the end of the UV lamp 340 and the bracket 360. Optionally, the UV lamp 340 may be secured to the shroud 312 through brackets or clamps that differ in size and shape than shown. As another example, the UV lamp 340 may be secured to the shroud 312 through adhesives, fasteners, and/or the like.

Figure 38 illustrates a perspective exploded view of the backpack assembly 304, according to an example of the present disclosure. The backpack assembly 304 includes a front wall 370 that couples to a rear shell 372, a base 374, and a top cap 376. An internal chamber 378 is defined between the front wall 370, the rear shell 372, the base 374, and the top cap 376. One or more batteries 380, such as rechargeable Lithium batteries, are contained within the internal chamber 378. An air generation sub-system 382 is also contained within the internal chamber 378. The air generation sub-system 382 is in fluid communication with an air tube within the hose 322 (shown in Figure 24, for example). The air generation sub-system 382 may include an airflow device, such as a vacuum generator, an air blower, and/or the like. The airflow device is configured to generate airflow to cool the UV lamp, draw air from the sanitizing head 306 into the backpack assembly 304 and out through an exhaust, draw or otherwise remove generated ozone away from the shroud 312, and/or the like.

One or more air filters 383, such as carbon filters, are within the backpack assembly 304. The air filters 383 are in communication with the air tube or other such delivery duct or line that routes air through the hose 322 and into the backpack assembly 304. The air filters 383 are configured to filter the air that is drawn into the backpack assembly 304 from the shroud 312. For example, the air filters 383 may be configured to remove, deactivate, or otherwise neutralize ozone.

The batteries 380 and/or a power supply within the backpack assembly 304 provide operating power for the UV lamp 340 of the sanitizing head 306 (shown in Figure 24, for example). The top wall 376 may be removably coupled to the front wall 370 and the rear shell 372. The top wall 376 may be removed to provide access to the batteries 380 (such as to remove and/or recharge the batteries), for example. Additional space may be provided within the backpack assembly 304 for storage of supplies, additional batteries, additional components, and/or the like. In at least one example, the front wall 370, the rear shell 372, the base 374, and the top cap 376 may be formed of fiberglass epoxy.

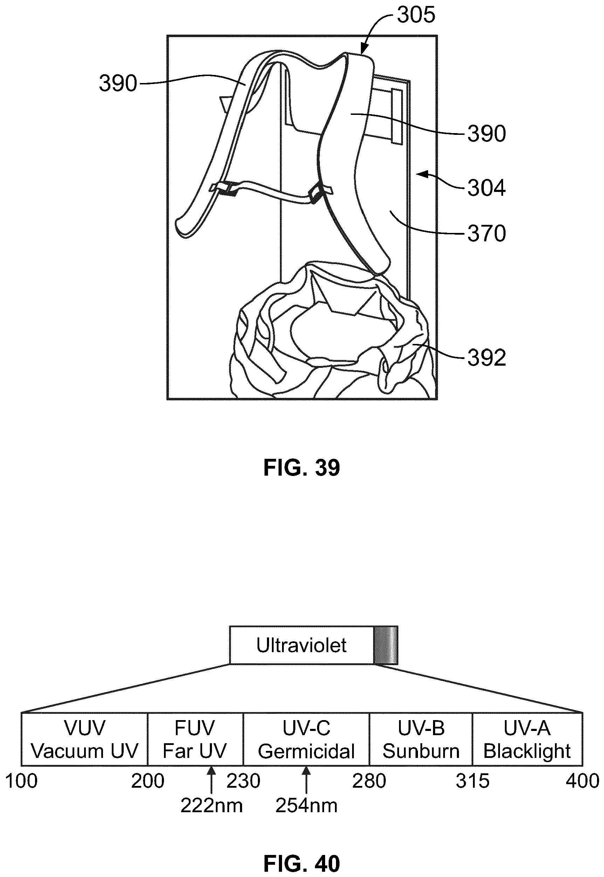

Figure 39 illustrates a perspective front view of the harness 305 coupled to the backpack assembly 304, according to an example of the present disclosure. The harness 305 may include shoulder straps 390 and/or a waist or hip belt or strap 392, which allow the individual to comfortably wear the backpack assembly 304.

Referring to Figures 23-39, in operation, the individual may walk through an area wearing the backpack assembly 304. When a structure to be sanitized is found, the individual may position grasp the handle 308 and position the sanitizing head 306 as desired, such as by extending and/or rotating the sanitizing head 306 relative to the handle 308. The individual may then engage an activation button on the handle 308, for example, to activate the UV lamp 340 to emit sanitizing UV light onto the structure. As the UV lamp 340 is activated, air 350 is drawn into the shroud 312 to cool the UV lamp 340, and divert any generated ozone into the backpack assembly 304, where it is filtered by the air filters 383.

The extendable wand assembly 302 allows the sanitizing head 306 to reach distant areas, such as over an entire set of three passenger seats, from a row within an internal cabin of a commercial aircraft.

Figure 40 illustrates an ultraviolet light spectrum. Referring to Figures 23-40, in at least one example, the sanitizing head 306 is configured to emit sanitizing UV light (through operation of the UV lamp 340) within a far UV spectrum, such as between 200 nm to 230 nm. In at least one example, the sanitizing head 306 emits sanitizing UV light having a wavelength of 222 nm. In at least one other example, the sanitizing head 306 is configured to emit sanitizing UV light within the UVC spectrum, such as between 230 nm to 280 nm. In at least one example, the sanitizing head 306 emits sanitizing UV light having a wavelength of 254 nm. Optionally, the sanitizing head 306 can be configured to emit UV light at wavelengths other than within the far UC spectrum or the UVC spectrum.

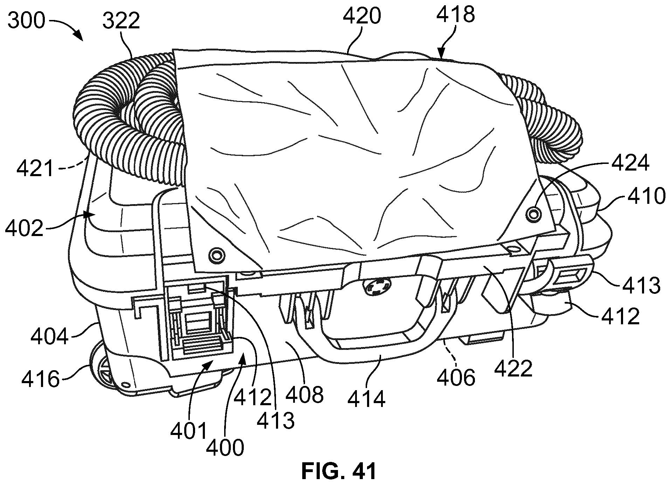

Figure 41 illustrates a perspective view of a portable sanitizing system 300, according to an example of the present disclosure. The portable sanitizing system 300 includes a case assembly 400 that is configured to store the wand assembly 402 (hidden from view in Figure 41) when the case assembly 400 is in a closed position, as shown in Figure 41.

The adapter modules 104 shown and described with respect to Figures 1-22 can be used with the sanitizing head of the wand assembly 402.

The case assembly 400 may be formed of plastic, for example. The case assembly 400 includes a main body 401, such as a shell, lower body portion, or the like. A cover 402, such as a lid, or upper body portion, is moveably coupled to the main body 401. For example, the cover 402 may be coupled to the main body 401 through a hinge that allows the cover 402 to be opened and closed relative to the main body 401.

The main body 401 includes a base 404 connected to a rear wall 406, lateral walls 408, and a top wall 410. The cover 402 is moveably coupled to a first lateral wall 408, such as through a hinge. One or more latches 412 are disposed on a second lateral wall 408, opposite from the first lateral wall 408. The latches 412 are configured to engage one or more reciprocal latch members 413 extending from the cover 402 to secure the cover 402 in the closed position. The latches 412 may be engaged by an individual to disengage the latch members 413 to allow the cover 402 to be pivoted into an open position.

A handle 414 is secured to the case assembly 400. For example, the handle 414 is pivotally secured to a lateral wall 408. The handle 414 is configured to be grasped by an individual so that the portable sanitizing system 300 may be carried. Optionally, the handle 414 may be secured to other portions of the case assembly 400, such as the top wall 410. In at least one example, the handle 414 may be configured to retract into the case assembly 400 into a fully retracted position, and extend out of (for example, telescope out of) the case assembly 400 into a fully extended position.

Casters 416 or other such wheels may be rotatably secured to a portion of the case assembly 400. For example, two casters 416 may be rotatably secured to the base 404 proximate to the rear wall 406. An individual may tilt the case assembly 400 so that the casters 416 contact a floor. In this manner, the individual may roll the portable sanitizing system 300 via the casters 416 (and optionally through a handle in an extended position from the top wall 410). Alternatively, the case assembly 400 may not include the casters 416.

The hose 322 may outwardly extend from the case assembly 400. In the closed position, when the wand assembly 302 is in a stowed position within the case assembly 400, the hose 322 may be coiled over the cover 402. A hose retainer 418 may secure the hose 322 in place on the cover 402. For example, the hose retainer 418 may include a flexible fabric sheet 420 that is secured to a first side 421 of the cover 402, and may removably secured to an opposite second side 422 of the cover 402, such as through one or more fastening members 424, such as hooks and loops, latches, clips, and/or the like. The hose retainer 418 is configured to secure the hose 322 on the cover 402 when the wand assembly 302 is within a storage chamber of the case assembly 400 and the cover 402 is in a closed position. Alternatively, the hose 322 may be contained within a storage chamber of the case assembly 400 when the wand assembly 302 is not in use. That is, the storage chamber may be sized and shaped to also contain the hose 322 when the wand assembly 302 is also within the storage chamber and the cover 402 is in the closed position.

The wand assembly 302 within the case assembly 400 in the closed position is protected from inadvertent engagement, bumping, and the like. That is, by storing the wand assembly 302 within the case assembly 400, which is closed, when the wand assembly 302 is not in use, the portable sanitizing system 300 protects the wand assembly 302 from potential damage, and increases the useful life of the wand assembly 302.

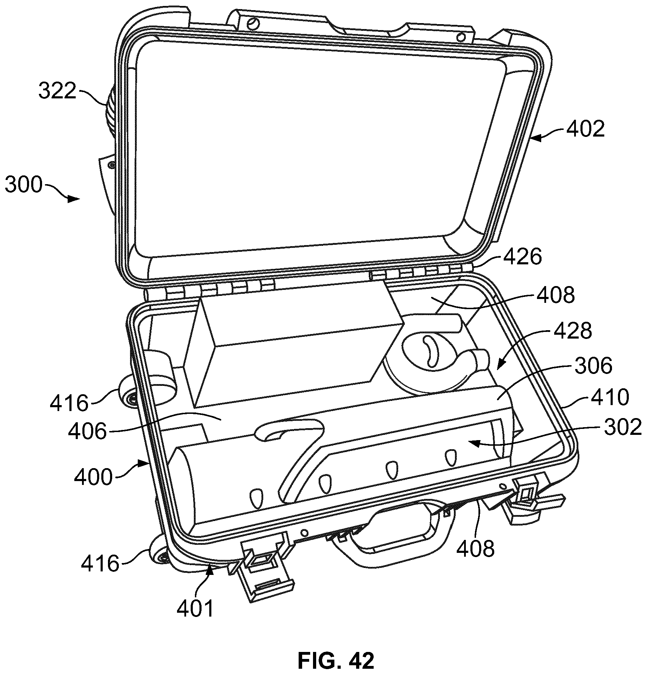

Figure 42 illustrates a perspective view of the portable sanitizing system 300 having the case assembly 400 in an open position, according to an example of the present disclosure. As shown, the cover 402 is opened via a hinge 426 that pivotally couples the cover 402 to the main body 401.

An internal or storage chamber 428 is defined between the base 404, the lateral walls 408, the rear wall 406, and the top wall 410 (and the cover 402, when closed). Various components of the portable sanitizing system 300 may be stored within the storage chamber 428. For example, the components within the backpack assembly 404, as described with respect to Figure 38, may be contained within the storage chamber 428.

For example, when not in use, the wand assembly 302 is contained within the storage chamber 428. Additionally, one or more batteries, such as rechargeable Lithium batteries, may be contained within the storage chamber 428.

An air generation sub-system (such as a cooling fan) may also be contained within the storage chamber 428. The air generation sub-system may be in fluid communication with an air tube within the hose 322. The hose 322 may be removably connected to the air generation sub-system. In at least one example, the hose 322 is configured to be coupled to and uncoupled from the wand assembly 302 and the air generation sub-system. That is, the hose 322 may be removably coupled to the wand assembly 302 and the air generation sub-system.

One or more air filters, such as carbon filters, may also be within the storage chamber 428. The air filters may be in communication with the air tube or other such delivery duct or line that routes air through the hose 422.

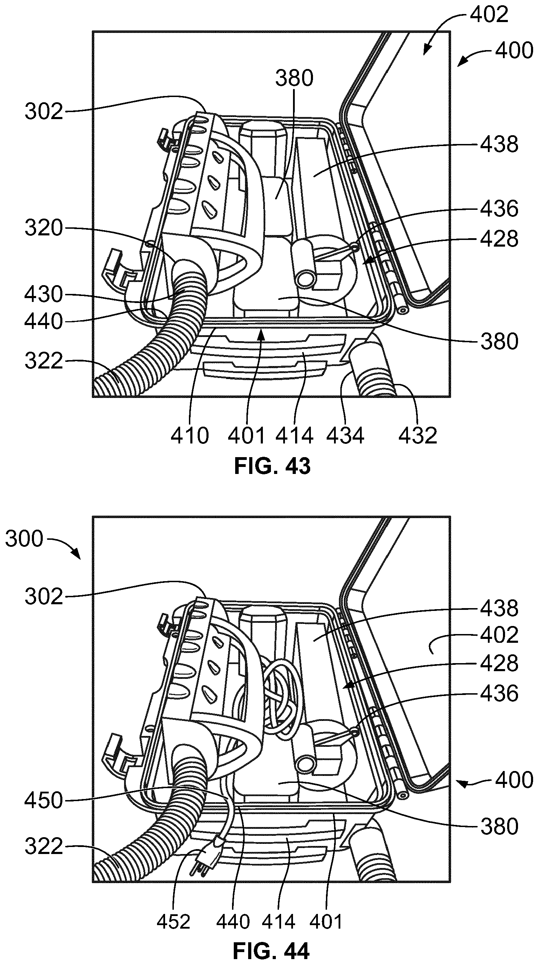

Figure 43 illustrates a perspective view of the portable sanitizing system 300 having the case assembly 400 in the open position, according to an example of the present disclosure. The wand assembly 302 is configured to be stowed in the storage chamber 428. When the wand assembly 302 is to be used, the cover 402 is opened, and a first end 430 of the hose 322 is coupled to the port 320 of the wand assembly 302. In at least one example, the hose 322 is configured to channel cooling air into the wand assembly 302, in order to cool the UV lamp 340 during activation.

A second end 432 of the hose 322 may be connected to a port 434 extending into and through a portion of the main body 401, such as through a portion of the top wall 410. The port 434 connects the hose 322 to an air generation sub-system, such as a cooling fan 436 that is within the storage chamber 428. The cooling fan 436 may be activated to generate cooling air that is delivered to the wand assembly 302 through the hose 322 (such as an air tube within the hose 322, or through an internal passage of the hose 322 itself).

One or more batteries 380 may also be stowed within the storage chamber 428. For example, three batteries 380 may be within the storage chamber 428.

A power supply 438 is also contained within the storage chamber 428. The power supply 438 may be coupled to the wand assembly 302 through a power cord (such as via a plug and receptacle fitting) to provide power to the wand assembly 302. Further, the power supply 438 may be configured to provide power to the batteries 380 (such as to recharge the batteries 380). The batteries 380 may be secured to the wand assembly 302 and provide power to the wand assembly 302, so that the wand assembly 302 may be used without connection to the power supply 438.

The cooling fan 436 couples to the hose 322 via the port 434. The cooling fan 436 may also include a diverter port that couples to an internal portion of the power supply 438. In this manner, cooling air may be delivered to both the hose 322 (and therefore the wand assembly 302), and the power supply 438, thereby providing cooling to both the wand assembly 302 and the power supply 438.

A hole 440 may be formed through a portion of the case assembly 400. For example, a hole 440 may be formed through a portion of the top wall 410 and sized and shaped to allow the hose 322 to pass therethrough. In this manner, the hose 322 may remain connected to the wand assembly 302 even when the wand assembly 302 is contained within the storage chamber 428 and the cover 402 is closed. Other portions of the hose 322 between the first end 430 and the second end 432 may be secured to the cover 402 by the hose retainer 418, as shown and described with respect to Figure 41.

As shown, the handle 414 may be secured to the top wall 410 of the main body 401. The handle 414 may be configured to retracted into and extend out of the main body 401. For example, the handle 414 may be a telescoping handle.

The wand assembly 302 is removably secured within the storage chamber 428. For example, the wand assembly 302 may be removably secured within the storage chamber 428 by one or more latches, clips, or via an interference fir with a conforming portion of the case assembly 400.

The power supply 438 may be fixed in position within the storage chamber 428. For example, the power supply 438 may be fixed in the storage chamber 428 by one or more fasteners, adhesives, or the like. Optionally, the power supply 438 may be secured in position by one or more latches, clips, or the like.

The batteries 380 may similarly be fixed position within the storage chamber 428. For example, the batteries 380 may be fixed in the storage chamber 428 by one or more fasteners, adhesives, or the like. Optionally, the batteries 380 may be secured in position by one or more latches, clips, or the like. In at least one other example, the batteries 380 may be removable, and configured to couple directly to the wand assembly 302 to provide power thereto.

Figure 44 illustrates a perspective view of the portable sanitizing system 300 having the case assembly 400 in the open position, according to an example of the present disclosure. A power cord 450 may also be stowed within the storage chamber 428. The power cord 450 is contained within the case assembly 400 when the cover 402 is closed and the portable sanitizing system 300 is moved when the wand assembly 302 is not being operated.

Optionally, the power cord 450 connects the power supply 438 to a source of power (such as a wall outlet). In addition to supply air to the wand assembly 302, the hose 322 also routes electrical cables and the like to the wand assembly 302 from the power supply 438 and the batteries 380.

Optionally, the hose 322 may not include electrical connections to the wand assembly 302. Instead, the wand assembly 302, the power cord 450 may plug into the wand assembly 302, via the plug 452, to supply power from the power supply 438 and/or the batteries 380. In this example, as the wand assembly 302 is operated, the plug 452 of the power cord 450 is connected to a reciprocal receptacle of the wand assembly 302. An opposite end of the power cord 450 is connected to the power supply 438 (and/or, a battery 380). The power cord 450 extends out of the case assembly 400 through the hole 440. Thus, the wand assembly 302 may be removed from the storage chamber 428 and connected to the hose 322 and the power cord 450, which extend through the hole 440. The cover 402 may then be closed, thereby securely retaining the power supply 438, the batteries 380, and the like within the storage chamber 428. The wand assembly 302 may then be activated, as it is powered via the power supply 438 or one or more of the batteries 380, and the closed case assembly 400 may be moved, such as via an individual grasping the handle 414 and rolling the case assembly 400 via the casters 416 (shown in Figures 41 and 42).

Further, the hole 440 also allows intake air to be drawn into the storage chamber 428, even when the cover 402 is closed over the main body 401. Accordingly, the cooling fan 436 is able to receive fresh air, even when the cover 402 is closed.

The power supply 438 may be configured to receive power from a standard power supply, such as a source of alternating current power. For example, the power supply 438 may connect to the source of alternating current power through a power cord. The power cord 450 connects to the wand assembly 452, and is configured to deliver power to the wand assembly 302 to operate the UV lamp 340 from power received from the power supply 438 and optionally the batteries 380. For example, when the power supply 438 is connected to a source of alternating current power, the wand assembly 302 is powered by the power supply 438. In the absence of such power, the wand assembly 302 may be powered by the batteries 380. For example, the wand assembly 302 receives power from the batteries 380 the power supply 438 is not plugged into a power outlet. If the power supply 438 is plugged into a power outlet, one or more relays in the power supply 438 switch over from the batteries 380 to alternating current power supply from the power outlet.

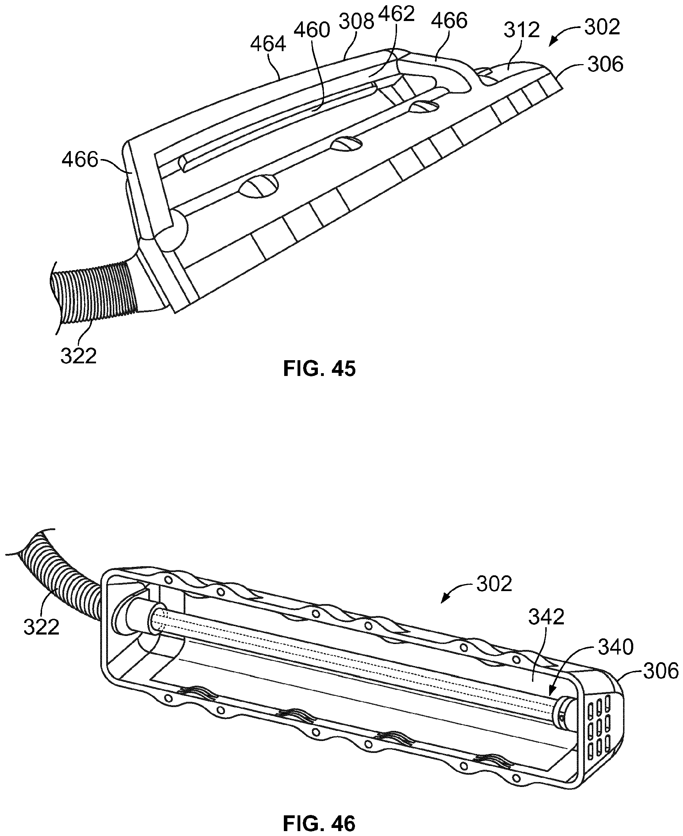

Figure 45 illustrates a perspective lateral view of the wand assembly 302, according to an example of the present disclosure. As shown, the handle 308 may be fixed in relation to the shroud 312. For example, the handle 308 may be integrally molded and formed with the shroud 312. The wand assembly 302 may be small and compact in order to fit in confined spaced, such as within a flight deck of an aircraft.

An activation trigger 460 is moveably coupled to the handle 308. For example, the activation trigger 460 may be secured to an underside 462 of a main beam 464 of the handle 308. The activation trigger 460 is configured to be selectively pressed and/or depressed to activate and deactivate the UV lamp 340 of the wand assembly 302, as desired.

The activation trigger 460 may be located anywhere along the length of the handle 308. The activation trigger 460 may be shaped differently than shown. Further, the activation trigger 460 may be smaller or larger than shown. As an example, the activation trigger 460 may be a circular button, instead of an elongated bar or beam, as shown. Also, optionally, the activation trigger 460 may be located on a top portion of the main beam 464, or on an extension beam 466, which spaces the handle 308 from the shroud 312. As another example, the activation trigger 460 may be located on a portion of the shroud 312.

Figure 46 illustrates a perspective bottom view of the wand assembly 302 of Figure 45. As shown, the reflector 342 is secured to an underside of the shroud 312.

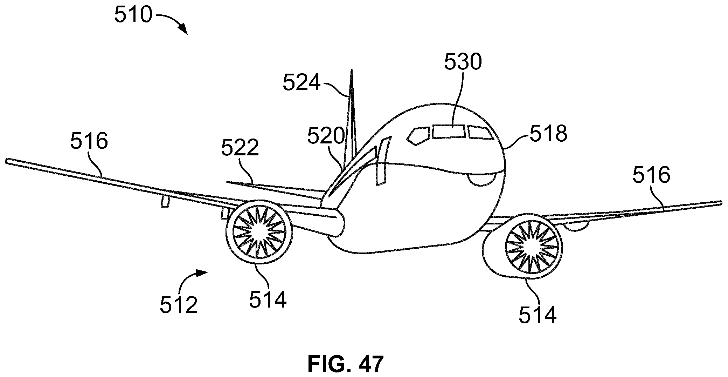

Figure 47 illustrates a perspective front view of an aircraft 510, according to an example of the present disclosure. The aircraft 510 includes a propulsion system 512 that includes engines 514, for example. Optionally, the propulsion system 512 may include more engines 514 than shown. The engines 514 are carried by wings 516 of the aircraft 510. In other examples, the engines 514 may be carried by a fuselage 518 and/or an empennage 520. The empennage 520 may also support horizontal stabilizers 522 and a vertical stabilizer 524.

The fuselage 518 of the aircraft 510 defines an internal cabin 530, which includes a flight deck or cockpit, one or more work sections (for example, galleys, personnel carry-on baggage areas, and the like), one or more passenger sections (for example, first class, business class, and coach sections), one or more lavatories, and/or the like. The sanitizing systems described herein can be used to sanitize surfaces, components, and the like within the internal cabin 530.

Optionally, instead of an aircraft, examples of the present disclosure may be used with various other vehicles, such as automobiles, buses, locomotives and train cars, watercraft, and the like. Further, examples of the present disclosure may be used with respect to fixed structures, such as commercial and residential buildings. Additionally, examples of the present disclosure can be used to sanitize surfaces within open air locations, such as stadiums, concert venues, open fields, and/or the like.

Further, the disclosure comprises examples according to the following examples :
In one example, an adaptable sanitizing method, may comprise:
removably coupling one or more adapter modules to a sanitizing head at a coupling interface, wherein sanitizing head includes an ultraviolet (UV) lamp configured to emit UV light, and wherein the one or more adapter modules are configured to provide a functionality in relation to the UV light emitted by the UV lamp.The functionality may comprise one of optical filtering, optical wavelength converting, fluid sanitizing, or object sanitizing.

The one or more adapter modules may comprise:
a first adapter module configured to perform a first unique functionality; and
a second adapter module configured to perform a second unique functionality that differs from the first unique functionality.

The adaptable sanitizing method may further comprise interchanging the first adapter module and the second adapter module in relation to the sanitizing head.

The first unique functionality may comprises one of optical filtering, optical wavelength converting, fluid sanitizing, or object sanitizing, and wherein the second unique functionality comprises another of optical filtering, optical wavelength converting, fluid sanitizing, or object sanitizing.

The coupling interface may be common to the first adaptable module and the second adapter module.

A wand assembly may include the sanitizing head.

The adaptable sanitizing method may further comprise coupling the wand assembly to a backpack assembly.

The adaptable sanitizing method may further comprise coupling the wand assembly is coupled to a case assembly.

In another example , a method may comprise:
coupling an optical filter to a shroud or frame, wherein the optical filter is configured to filter ultraviolet (UV) light emitted by a UV lamp; and
providing an adapter module by said coupling, wherein the adapter module is configured to removably couple to a sanitizing head having the UV lamp at a coupling interface.

The adapter module may be distinct from the sanitizing head.

The optical filter may bea 230 nanometer low pass filter.

Said coupling may comprise securing a panel to the shroud or the frame.

The method may further comprise forming the shroud or the frame and the optical filter of the same light filtering material.

The method may further comprise forming the optical filter from silicon.

The method may further comprise one or both of:
doping the optical filter with a metallic coating; or
etching a particular pattern into or onto the optical filter.

The coupling interface is common to the adaptable module and another adapter module that differs from the adaptable module.

As described herein, examples of the present disclosure provide systems and methods for adapting a UV light sanitizing system. Further, examples of the present disclosure provide UV light sanitizing systems and methods that can be adapted to and used with respect to various different applications.

While various spatial and directional terms, such as top, bottom, lower, mid, lateral, horizontal, vertical, front and the like can be used to describe examples of the present disclosure, it is understood that such terms are merely used with respect to the orientations shown in the drawings. The orientations can be inverted, rotated, or otherwise changed, such that an upper portion is a lower portion, and vice versa, horizontal becomes vertical, and the like.

As used herein, a structure, limitation, or element that is "configured to" perform a task or operation is particularly structurally formed, constructed, or adapted in a manner corresponding to the task or operation. For purposes of clarity and the avoidance of doubt, an object that is merely capable of being modified to perform the task or operation is not "configured to" perform the task or operation as used herein.

It is to be understood that the above description is intended to be illustrative, and not restrictive. For example, the above-described examples (and/or aspects thereof) can be used in combination with each other.

While the dimensions and types of materials described herein are intended to define the parameters of the various examples of the disclosure, the examples are by no means limiting and are exemplary examples. Many other examples will be apparent to those of skill in the art upon reviewing the above description.

In the appended claims and the detailed description herein, the terms "including" and "in which" are used as the plain-English equivalents of the respective terms "comprising" and "wherein." Moreover, the terms "first," "second," and "third," etc. are used merely as labels, and are not intended to impose numerical requirements on their objects.

This written description uses examples to disclose the various examples of the disclosure, including the best mode, and also to enable any person skilled in the art to practice the various examples of the disclosure, including making and using any devices or systems and performing any incorporated methods. The patentable scope of the various examples of the disclosure is defined by the claims.

## Claims

1. An adaptable sanitizing system (100), comprising:
a sanitizing head (102) including an ultraviolet (UV) lamp (108) configured to emit UV light;
one or more adapter modules (104) configured to removably couple to the sanitizing head (102) at a coupling interface (106),
the one or more adapter modules (104) being configured to provide a functionality of in relation to the UV light emitted by the UV lamp (108),
the one or more adapter modules (104) comprising:
a first adapter configured to perform a first unique functionality of optical filtering, and
a second adapter module configured to perform a second unique functionality of fluid sanitizing,
wherein said second adapter module comprises:
a shroud that removably couples to the sanitizing head at the coupling interface; and
a fluid passage in fluid communication with an inlet and an outlet within the shroud, the fluid passage being configured to receive fluid through the inlet and pass the fluid out of the outlet, the UV lamp being configured to emit the UV light into the fluid passage as the fluid passes through the fluid passage between the inlet and the outlet.

2. The adaptable sanitizing system (100) of claim 1, wherein the first adapter module and the second adapter module are interchangeable in relation to the sanitizing head (102).

3. The adaptable sanitizing system (100) of claim 1 or 2, wherein the coupling interface (106) is common to the first adaptable module and the second adapter module.

4. The adaptable sanitizing system (100) of any of claims 1 to 3, wherein the fluid passage is defined by internal surfaces of the shroud and wherein the internal surfaces of the shroud are reflective.

5. The adaptable sanitizing system (100) of any of claims 1 to 4, wherein the second adapter module further comprises:
a particulate filter disposed within the fluid passage; and
an ozone filter disposed within the fluid passage.

6. The adaptable sanitizing system (100) of any of claims 1 to 5, wherein the second adapter module comprises one or more glass tubes within the shroud, wherein the one or more glass tubes define the fluid passage, and wherein the one or more glass tubes comprise a plurality of straight segments coupled to one or more bends.

7. The adaptable sanitizing system (100) of any of claims 1 to 6, wherein the second adapter module further comprises a blower disposed within the shroud.

8. The adaptable sanitizing system (100) of any of claims 1 to 7, wherein the second adapter module further comprises one or both of a valve or a pump proximate to the inlet, wherein one or both of the valve or the pump are configured to control flow of liquid through the fluid passage.

9. The adaptable sanitizing system (100) of any of claims 1 to 8, further comprising a wand assembly that includes the sanitizing head (102).

10. The adaptable sanitizing system (100) of claim 9, further comprising a backpack assembly (304) and wherein the wand assembly is removably coupled to a backpack assembly.

11. The adaptable sanitizing system (100) of claim 9, further comprising a case assembly configured to be opened and closed, and wherein the wand assembly is stored within said case assembly.

12. The adaptable sanitizing system (100) of any of claims 1 to 11, wherein the first adapter module comprises :
a shroud (180) or frame that is configured to removably couple to the sanitizing head (102) at a coupling interface (106); and
an optical filter coupled to the shroud (180) or the frame,
wherein the optical filter is configured to filter the UV light emitted by the UV lamp (108)

13. The adaptable sanitizing system (100) of claim 12, wherein the optical filter is a 230 nanometer low pass filter.

14. The adaptable sanitizing system (100) of claim 12 or 13, wherein the optical filter comprises a panel secured to the shroud (180) or the frame.

15. The adaptable sanitizing system of any of claim 12 to 14, wherein the optical filter is formed from silicon, and wherein the optical filter is one or both of doped with a metallic coating or etched in a particular pattern to filter the UV light at a predetermined wavelength.

## Patentansprüche

1. Anpassungsbares Desinfektionssystem (100), das aufweist:
einen Desinfektionskopf (102), der eine Ultraviolett-(UV)-Lampe (108) aufweist, die konfiguriert ist, UV-Licht zu emittieren;
ein oder mehrere Adaptermodule (104), die konfiguriert sind, an einer Kopplungsschnittstelle (106) abnehmbar mit dem Desinfektionskopf (102) zu koppeln,
wobei das eine oder die mehreren Adaptermodule (104) konfiguriert sind, eine Funktionalität in Bezug auf das von der UV-Lampe (108) emittierte UV-Licht bereitzustellen,
wobei das eine oder die mehreren Adaptermodule (104) aufweisen:
einen ersten Adapter, der konfiguriert ist, eine erste besondere Funktionalität der optischen Filterung auszuführen, und
ein zweites Adaptermodul, das konfiguriert ist, eine zweite besondere Funktionalität der Fluiddesinfektion auszuführen,
wobei das zweite Adaptermodul aufweist:
eine Verkleidung, die abnehmbar mit dem Desinfektionskopf an der Kopplungsschnittstelle koppelt; und
einen Fluidkanal in Fluidverbindung mit einem Einlass und einem Auslass innerhalb der Verkleidung, wobei der Fluidkanal konfiguriert ist, Fluid durch den Einlass aufzunehmen und das Fluid aus dem Auslass herauszuleiten, wobei die UV-Lampe konfiguriert ist, das UV-Licht in den Fluidkanal zu emittieren, wenn das Fluid durch den Fluidkanal zwischen dem Einlass und dem Auslass hindurchtritt.

2. Anpassungsbares Desinfektionssystem (100) nach Anspruch 1, wobei das erste Adaptermodul und das zweite Adaptermodul in Bezug auf den Desinfektionskopf (102) austauschbar sind.

3. Anpassungsbares Desinfektionssystem (100) nach Anspruch 1 oder 2, wobei die Kopplungsschnittstelle (106) dem ersten Adaptermodul und dem zweiten Adaptermodul gemeinsam ist.

4. Anpassungsbares Desinfektionssystem (100) nach einem der Ansprüche 1 bis 3, wobei der Fluidkanal durch Innenflächen der Verkleidung definiert ist und wobei die Innenflächen der Verkleidung reflektierend sind.

5. Anpassungsbares Desinfektionssystem (100) nach einem der Ansprüche 1 bis 4, wobei das zweite Adaptermodul ferner aufweist:
einen Partikelfilter, der in dem Fluidkanal angeordnet ist; und
einen Ozonfilter, der in dem Fluidkanal angeordnet ist.

6. Anpassungsbares Desinfektionssystem (100) nach einem der Ansprüche 1 bis 5, wobei das zweite Adaptermodul ein oder mehrere Glasröhren in der Verkleidung aufweist, wobei die eine oder mehreren Glasröhren den Fluidkanal definieren und wobei die eine oder mehreren Glasröhren mehrere gerade Segmente aufweisen, die mit einer oder mehreren Biegungen gekoppelt sind.

7. Anpassungsbares Desinfektionssystem (100) nach einem der Ansprüche 1 bis 6, wobei das zweite Adaptermodul ferner ein in der Verkleidung angeordnetes Gebläse aufweist.

8. Anpassungsbares Desinfektionssystem (100) nach einem der Ansprüche 1 bis 7, wobei das zweite Adaptermodul ferner eines oder beides eines Ventils oder einer Pumpe in der Nähe des Einlasses aufweist, wobei eines oder beides des Ventils oder der Pumpe konfiguriert ist oder sind, den Fluidstrom durch den Fluidkanal steuern.

9. Anpassungsbares Desinfektionssystem (100) nach einem der Ansprüche 1 bis 8, das ferner eine Stabanordnung aufweist, die den Desinfektionskopf (102) aufweist.

10. Anpassungsbares Desinfektionssystem (100) nach Anspruch 9, das ferner eine Rucksackanordnung (304) aufweist, wobei die Stabanordnung abnehmbar mit einer Rucksackanordnung gekoppelt ist.

11. Anpassungsbares Desinfektionssystem (100) nach Anspruch 9, das ferner eine Gehäuseanordnung aufweist, die konfiguriert ist, geöffnet und geschlossen zu werden, und wobei die Stabanordnung in der Gehäuseanordnung aufbewahrt wird.

12. Anpassungsbares Desinfektionssystem (100) nach einem der Ansprüche 1 bis 11, wobei das erste Adaptermodul aufweist:
eine Verkleidung (180) oder einen Rahmen, die oder der konfiguriert ist, an einer Kopplungsschnittstelle (106) abnehmbar mit dem Desinfektionskopf (102) gekoppelt zu werden; und
einen optischen Filter, der mit der Verkleidung (180) oder dem Rahmen gekoppelt ist,
wobei der optische Filter konfiguriert ist, das von der UV-Lampe (108) emittierte UV-Licht zu filtern.

13. Anpassungsbares Desinfektionssystem (100) nach Anspruch 12, wobei der optische Filter ein 230 Nanometer Tiefpassfilter ist.

14. Anpassungsbares Desinfektionssystem (100) nach Anspruch 12 oder 13, wobei der optische Filter eine Platte aufweist, die an der Verkleidung (180) oder dem Rahmen befestigt ist.

15. Anpassungsbares Desinfektionssystem nach einem der Ansprüche 12 bis 14, wobei der optische Filter aus Silizium ausgebildet ist und wobei der optische Filter mit einer metallenen Beschichtung dotiert und/oder in einem bestimmten Muster geätzt ist, um das UV-Licht bei einer vorbestimmten Wellenlänge zu filtern.

## Revendications

1. Système de désinfection adaptable (100), comprenant :
une tête de désinfection (102) comportant une lampe à ultraviolet (UV) (108) configurée pour émettre une lumière UV ;
un ou plusieurs modules adaptateurs (104) configurés pour se coupler de manière amovible à la tête de désinfection (102) au niveau d'une interface de couplage (106),
les un ou plusieurs modules adaptateurs (104) étant configurés pour fournir une fonctionnalité par rapport à la lumière UV émise par la lampe à UV (108),
les un ou plusieurs modules adaptateurs (104) comprenant :
un premier adaptateur configuré pour réaliser une première fonctionnalité unique de filtrage optique, et
un second module adaptateur configuré pour réaliser une seconde fonctionnalité unique de désinfection de fluide,
dans lequel ledit second module adaptateur comprend :
un épaulement qui se couple de manière amovible à la tête de désinfection au niveau de l'interface de couplage ; et
un passage de fluide en communication fluidique avec une entrée et une sortie à l'intérieur de l'épaulement, le passage de fluide étant configuré pour recevoir un fluide à travers l'entrée et faire passer le fluide hors de la sortie, la lampe à UV étant configurée pour émettre la lumière UV dans le passage de fluide lorsque le fluide passe à travers le passage de fluide entre l'entrée et la sortie.

2. Système de désinfection adaptable (100) selon la revendication 1, dans lequel le premier module adaptateur et le second module adaptateur sont interchangeables par rapport à la tête de désinfection (102).

3. Système de désinfection adaptable (100) selon la revendication 1 ou 2, dans lequel l'interface de couplage (106) est commune au premier module adaptateur et au second module adaptateur.

4. Système de désinfection adaptable (100) selon l'une quelconque des revendications 1 à 3, dans lequel le passage de fluide est défini par des surfaces internes de l'épaulement et dans lequel les surfaces internes de l'épaulement sont réfléchissantes.

5. Système de désinfection adaptable (100) selon l'une quelconque des revendications 1 à 4, dans lequel le second module adaptateur comprend en outre :
un filtre à particules disposé dans le passage de fluide ; et
un filtre à ozone disposé dans le passage de fluide.

6. Système de désinfection adaptable (100) selon l'une quelconque des revendications 1 à 5, dans lequel le second module adaptateur comprend un ou plusieurs tubes en verre à l'intérieur de l'épaulement, dans lequel les un ou plusieurs tubes en verre définissent le passage de fluide, et dans lequel les un ou plusieurs tubes en verre comprennent une pluralité de segments rectilignes couplés à un ou plusieurs coudes.

7. Système de désinfection adaptable (100) selon l'une quelconque des revendications 1 à 6, dans lequel le second module adaptateur comprend en outre une soufflante disposée à l'intérieur de l'épaulement.

8. Système de désinfection adaptable (100) selon l'une quelconque des revendications 1 à 7, dans lequel le second module adaptateur comprend en outre l'une ou les deux d'une vanne ou d'une pompe à proximité de l'entrée, dans lequel l'une ou les deux de la vanne ou de la pompe sont configurées pour réguler le débit de liquide à travers le passage de fluide.

9. Système de désinfection adaptable (100) selon l'une quelconque des revendications 1 à 8, comprenant en outre un ensemble lance qui comporte la tête de désinfection (102).

10. Système de désinfection adaptable (100) selon la revendication 9, comprenant en outre un ensemble sac à dos (304) et dans lequel l'ensemble lance est couplé de manière amovible à un ensemble sac à dos.

11. Système de désinfection adaptable (100) selon la revendication 9, comprenant en outre un ensemble boîtier configuré pour être ouvert et fermé, et dans lequel l'ensemble lance est rangé au sein dudit ensemble boîtier.

12. Système de désinfection adaptable (100) selon l'une quelconque des revendications 1 à 11, dans lequel le premier module adaptateur comprend :
un épaulement (180) ou un cadre qui est configuré pour se coupler de manière amovible à la tête de désinfection (102) au niveau d'une interface de couplage (106) ; et
un filtre optique couplé à l'épaulement (180) ou au cadre,
dans lequel le filtre optique est configuré pour filtrer la lumière UV émise par la lampe à UV (108).

13. Système de désinfection adaptable (100) selon la revendication 12, dans lequel le filtre optique est un filtre passe-bas de 230 nanomètres.

14. Système de désinfection adaptable (100) selon la revendication 12 ou 13, dans lequel le filtre optique comprend un panneau fixé à l'épaulement (180) ou au cadre.

15. Système de désinfection adaptable selon l'une quelconque des revendications 12 à 14, dans lequel le filtre optique est formé de silicium, et dans lequel le filtre optique est l'un ou les deux parmi un filtre dopé d'un revêtement métallique ou gravé selon un motif particulier pour filtrer la lumière UV à une longueur d'onde prédéterminée.
